# EUROPEAN PATENT APPLICATION

(11) **EP 1 375 654 A2**
(43) Date of publication of application: **02.01.2004**
(21) Application number: 03014030.5
(22) Date of filing: 02.11.1999
(51) Int. Cl.: C12N 15/00, A01K 67/027, C07K 14/81

(54) **Transgenic and cloned mammals**

(30) Priority: 02.11.1998 US 106728; 22.04.1999 US 298508; 23.04.1999 US 298971; 26.04.1999 US 131328
(62) Divisional of application: 99971451.2
(71) Applicant: GTC Biotherapeutics, Inc., Framingham, MA 01702 (US)
(72) Inventor: Echelard, Yann, Jamaica Plains, MA 02131 (US)
(74) Representative: Ruffles, Graham Keith

(57) **Abstract**

The invention features methods of making cloned and transgenic non-human mammals, for instance goats. The methods include making a somatic cell line, for instance a transgenic somatic cell line, which can be used as a donor cell, methods of producing a cloned or transgenic non-human mammal by introducing the genome of a somatic cell into an enucleated oocyte, preferably a naturally matured oocyte in the metaphase II stage of meiotic cell division, to form a reconstructed embryo, and methods of transferring the reconstructed embryo. The invention also includes cell lines, reconstructed embryos and cloned or transgenic non-human mammals.

## Description

This application claims the benefit of a previously filed Provisional Application No. 60/106,728, filed November 2, 1998, Provisional Application No. 60/131,328, filed April 26, 1999, U.S Serial No.: 09/298,971 filed April 23, 1999, and U.S. Serial No.: 09/298,508, filed April 22, 1999, all of which are hereby incorporated by reference.

### Background of the Invention

The ability to modify animal genomes through transgenic technology has opened new avenues for medical applications. By targeting the expression of biomedical proteins to the mammary gland of large farm animals, low-cost production of high quantities of valuable therapeutic proteins is now possible. Houdebine (1995) *Reprod. Nutr. Dev.* 35:609-617; Maga et al. (1995) *Bio*/*Technology,* 13:1452-1457; Echelard (1996) *Curr.Op.Biotechnol.* 7:536-540; Young et al. (1997) *BioPharm.* 10:34-38. Although the total sales for the top fifteen biopharmaceuticals in 1996 were $7.5 billion, expectations are that this number will continue to rise in the future. *Med. Ad News* 16:30. Transgenic technology is applicable and attractive for proteins that, whether due to high unit dosage requirements, frequency of administration, or large patient populations, are needed in high volume, and also to complex proteins that are difficult to produce in commercially viable quantities using traditional cell culture methods. In addition, the production of human pharmaceuticals in the milk of transgenic farm animals solves many of the problems associated with microbial bioreactors, e.g., lack of post-translational modifications, improper folding, high purification costs, or animal cell bioreactors, e.g., high capital costs, expensive culture media, low yields.

Dairy goats are ideal for transgenic production of therapeutic recombinant proteins. Their average milk output is 600-800 liters per lactation. With herds of a manageable size and at concentrations of approximately 1-5 grams/liter reproducibly achieved with various animal models, yields of transgenic protein to obtain 1-300 kg of purified product per year are achievable. Gordon et al. (1987) *Bio*/*Technology* 5:1183-1187; Meade et al. (1990) *Bio*/*Technology* 8:443-446; Ebert et al. (1991) *Bio*/*Technology* 9:835-838; Simons et al. (1987) *Nature* 328:530-532; Wright et al. (1991) *Bio*/*Technology*9:801-834; Velander et al. (1992) *Proc Natl Acad Sci USA* 89:12003-120007; Hansson et al. (1994) *J Biol Chem.* 269:5358-5363; Hurwitz et al. (1994) *Transgenic Res*. 3:365-375. This represents the low to middle range of the high volume protein category and quantities that would be required for the majority of biopharmaceuticals currently under development. Moreover, the goat generation interval, i.e., gestation, growth to sexual maturity and gestation, is 18 months as compared to almost three years for cows. This period permits expansion of the production herds within the time frame needed for the regulatory approval of the transgenically-produced therapeutic proteins. Finally, the much lower incidence of scrapie in goats (only 7 cases ever reported in the U.S.) relative to sheep, which have identical reproductive performance, and lower lactation output, makes goats better candidates for the production of therapeutic proteins.

Currently, there are very few reliable methods of producing transgenic goats. One such method is pronuclear microinjection. Using pronuclear microinjection methods, transgene integration into the genetic make up occurs in 1-3% of all the microinjected embryos. Ebert et al. (1993) *Theriogenology,* 39:121-135.

In 1981, it was reported that mouse embryonic stem cells can be isolated, propagated *in vivo,* genetically modified and, ultimately, can contribute to the germline of a host embryo. Evans et al. (1981) *Nature* 292:154-156; Martin *(1981)Proc Natl Acad Sci USA* 78:7634-7638; Bradley et al. (1984) *Nature* 309:255-256. Since then, murine embryonic stem cells have been extensively exploited in developmental and genetic studies to modify, e.g., delete, replace, mutate, single targeted genes. Mansour et al. (1988) *Nature 336:348-352;* McMahon et al. (1990) *Cell* 62:1073-1085; recently reviewed in: Bronson et al. *(1994) J. Biol. Chem.* 269:27155-27158; Rossant, et al. *(1995) Nat. Med.* 6:592-594. Although extensive studies in the mouse have clearly indicated the utility of these elegant and powerful techniques, successful application of embryonic cell technology has been conclusively reported only in the mouse.

A need exists, however, for methods for obtaining cloned and transgenic animals such as goats.

### Summary of the Invention

The present invention is based, at least in part, on the discovery that cloned and transgenic mammals, e.g., cloned and transgenic goats, can be produced by introduction of a somatic cell chromosomal genome into a functionally enucleated oocyte with simultaneous activation. The functionally enucleated oocyte can be activated or nonactivated. In one embodiment, a nonactivated functionally enucleated oocyte (e.g., a caprine oocyte at metaphase II stage) is fused (e.g., by electrofusion) with a donor somatic cell (e.g., a caprine somatic cell) and simultaneously activated with fusion. In another embodiment, an activated functionally enucleated oocyte (e.g., a naturally matured caprine oocyte at telophase stage) is fused (e.g., by electrofusion) with a donor somatic cell (e.g., a caprine somatic cell) and simultaneously activated with fusion.

The use somatic cell lines, e.g., recombinant primary somatic cell lines, for nuclear transfer of transgenic nuclei dramatically increases the efficiency of production of transgenic animals, e.g., up to 100%, if the animals are made by the methods described herein. It also solves the initial mosaicism problem as each cell in the developing embryo contains the transgene. In addition, using nuclear transfer from transgenic cell lines to generate transgenic animals, e.g., transgenic goats, permits an accelerated scale up of a specific transgenic line. For example, a herd can be scaled up in one breeding season.

The generation of transgenic animals, e.g., transgenic goats, by nuclear transfer with somatic cells has the additional benefit of allowing genetic manipulations that are not feasible with traditional microinjection approaches. For example, nuclear transfer with somatic cells allows the introduction of specific mutations, or even the targeting of foreign genes directed to specific sites in the genome solving the problem of integration position effect. Homologous recombination in the donor somatic cells can "knock-out" or replace the endogenous protein, e.g., a endogenous goat protein, to lower purification costs of heterologous proteins expressed in milk and help to precisely adjust the animal bioreactors.

In general, the invention features a method of providing a cloned non-human mammal, e.g., a cloned goat. The methods below are described for goats, but can be applied for any non-human mammal. The method includes: introducing a caprine genome from a caprine somatic cell into a caprine oocyte, preferably a naturally matured telophase oocyte, to form a reconstructed embryo; and allowing the reconstructed embryo to develop into a goat, e.g., by introducing the reconstructed embryo into a recipient doe, thereby providing a goat.

In one embodiment, the nucleus of the caprine somatic cell is introduced into the caprine oocyte, e.g., by direct nuclear injection or by fusion, e.g., electrofusion, of the somatic cell with the oocyte.

In preferred embodiment, the goat develops from the reconstructed embryo. In another embodiment, the goat is a descendant of a goat which developed from the reconstructed embryo.

In a preferred embodiment: the somatic cell is non-quiescent (e.g., the cell is activated), e.g., the somatic cell is in G₁ stage. In another preferred embodiment, the somatic cell is quiescent (e.g., the cell is arrested), e.g., the somatic cell is in G₀ stage. In a preferred embodiment, the somatic cell is an embryonic somatic cell, e.g., the somatic cell is an embryonic fibroblast. The somatic cell can be any of: a fibroblast (e.g., a primary fibroblast), a muscle cell (e.g., a myocyte), a cumulus cell, a neural cell or a mammary cell.

In a preferred embodiment, the oocyte is a functionally enucleated oocyte, e.g., an enucleated oocyte. In a preferred embodiment, the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is activated prior to or simultaneously with the introduction of the genome. In another preferred embodiment, the oocyte and somatic cell are synchronized, e.g., both the oocyte and somatic cell are activated or both the oocyte and somatic cell are arrested.

In a preferred embodiment, the method further includes mating the goat which develops from the reconstructed embryo with a second goat. A second goat can be a normal goat, a second goat which develops from a reconstructed embryo or is descended from a goat which developed from a reconstructed embryo or a second goat developed from a reconstructed embryo, or descended from a goat which developed from a reconstructed embryo, which was formed from genetic material from the same animal, an animal of the same genotype, or same cell line, which supplied the genetic material for the first goat. In a preferred embodiment, a first transgenic goat which develops from the reconstructed embryo can be mated with a second transgenic goat which developed from a reconstructed embryo and which contains a different transgene that the first transgenic goat.

In a preferred embodiment, the goat is a male goat. In other preferred embodiments, the goat is a female goat. A female goat can be induced to lactate and milk can be obtained from the goat.

In a preferred embodiment: a product, e.g., a protein, e.g., a recombinant protein, e.g., a human protein, is recovered from the goat; a product, e.g., a protein, e.g., a human protein, is recovered from the milk, urine, hair, blood, skin or meat of the goat.

In another aspect, the invention features a method of providing a transgenic non-human mammal, e.g., a transgenic goat. The methods below are described for goats, but can be applied for any non-human mammal. The method includes: introducing a genetically engineered caprine genome of a caprine somatic cell into a caprine oocyte, preferably a naturally matured telophase oocyte, to form a reconstructed embryo; and allowing the reconstructed embryo to develop into a goat, e.g., by introducing the reconstructed embryo into a recipient doe, thereby providing a transgenic goat.

In one embodiment, the nucleus of the genetically engineered caprine somatic cell is introduced into the caprine oocyte, e.g., by direct nuclear injection or by fusion, e.g., electrofusion, of the somatic cell with the oocyte.

In preferred embodiment, the goat develops from the reconstructed embryo. In another embodiment, the goat is a descendant of a goat which developed from the reconstructed embryo.

In a preferred embodiment: the somatic cell is non-quiescent (e.g., the cell is activated), e.g., the somatic cell is in G₁ stage. In another preferred embodiment, the somatic cell is quiescent (e.g., the cell is arrested), e.g., the somatic cell is in G₀ stage. In a preferred embodiment, the somatic cell is an embryonic somatic cell, e.g., the somatic cell is an embryonic fibroblast. A somatic cell can be any of: a fibroblast (e.g., a primary fibroblast), a muscle cell (e.g., a myocyte), a cumulus cell, a neural cell or a mammary cell.

In a preferred embodiment, a transgenic sequence has been introduced into the somatic cell; the somatic cell is from a cell line, e.g., a primary cell line; the somatic cell is from a cell line and a transgenic sequence has been inserted into the cell.

In a preferred embodiment, the oocyte is a functionally enucleated oocyte, e.g., an enucleated oocyte.

In a preferred embodiment, the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is activated prior to or simultaneously with the introduction of the genetically engineered genome. In another preferred embodiment, the oocyte and somatic cell are synchronized, e.g., both the oocyte and the somatic cell are activated or both the oocyte and somatic cell are arrested.

In a preferred embodiment, the method further includes mating the transgenic goat which develops from the reconstructed embryo with a second goat. The second goat can be a normal goat, a second goat which develops from a reconstructed embryo or is descended from a goat which developed from a reconstructed embryo or a second goat developed from a reconstructed embryo, or descended from a goat which developed from a reconstructed embryo, which was formed from genetic material from the same animal, an animal of the same genotype, or same cell line, which supplied the genetic material for the first goat. In a preferred embodiment, a first transgenic goat which develops from the reconstructed embryo can be mated with a second transgenic goat which developed from a reconstructed embryo and which contains a different transgene than the first transgenic goat.

In a preferred embodiment, the goat is a male goat. In other preferred embodiments the goat is a female goat. A female goat can be induced to lactate and milk can be obtained from the goat.

In a preferred embodiment: a product, e.g., a protein, e.g., a recombinant protein, e.g., a human protein, is recovered from the goat; a product, e.g., a protein, e.g., a human protein, is recovered from the milk, urine, hair, blood, skin or meat of the goat.

In a preferred embodiment, the caprine genome of the somatic cell includes a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest such as a protein, polypeptide or peptide. A protein can be any of: a hormone, an immunoglobulin, a plasma protein, and an enzyme. The transgenic sequence can encode any protein whose expression in the transgenic goat is desired including, but not limited to, any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, elythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the caprine genome includes a heterologous transgenic sequence under the control of a promoter, e.g., a caprine promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be any of: a casein promoter, a beta lactoglobulin promoter, a whey acid protein promoter and a lactalbumin promoter.

In another aspect, the invention features a method of making or producing a non-human mammal, e.g., a goat, e.g., a cloned or transgenic goat. The methods below are described for goats, but can be applied for any non-human mammal. The method includes fusing, e.g., by electrofusion, a caprine somatic cell, e.g., a caprine somatic cell capable of expressing a transgenic protein, with an enucleated caprine oocyte, preferably a naturally matured telophase oocyte, to obtain a reconstructed embryo; activating the reconstructed embryo; transferring the embryo into a recipient doe; and allowing the embryo to develop into a goat.

In a preferred embodiment, the goat develops from the reconstructed embryo. In another embodiment, the goat is a descendant of a goat which developed from the reconstructed embryo.

In a preferred embodiment, the somatic cell is an embryonic somatic cell. A somatic cell can be any of: a fibroblast (e.g., a primary fibroblast), a muscle cell (e.g., a myocyte), a cumulus cell, a neural cell or a mammary cell. In a preferred embodiment, the somatic cell is a non-quiescent cell (e.g., the cell is activated), e.g., the somatic cell is in G₁ stage, e.g., in G₁ prior to START. In another preferred embodiment, the somatic cell is a quiescent cell (e.g., the cell is arrested), e.g., the somatic cell is in G₀ stage.

In a preferred embodiment, the oocyte is in metaphase II. Alternatively, the oocyte is in telophase. In either embodiment, the oocyte is activated prior to or simultaneously with the introduction of the genome. In a preferred embodiment, the oocyte is obtained using an *in vivo* protocol; the oocyte is obtained using an *in vivo* protocol to obtain an oocyte which is in a desired stage of the cell cycle, e.g., metaphase II or telophase. In a preferred embodiment, the oocyte and somatic cell are synchronized, e.g., both the oocyte and somatic cell are activated or both the oocyte and somatic cell are arrested.

In a preferred embodiment: a transgenic sequence has been introduced into the somatic cell; the somatic cell is from a cell line, e.g., a primary cell line; the somatic cell is from a cell line and a transgenic sequence has been inserted into the cell.

In a preferred embodiment, the method further includes mating the goat which develops from the reconstructed embryo with a second goat. A second goat can be a normal goat, a second goat which develops from a reconstructed embryo or is descended from a goat which developed from a reconstructed embryo or a second goat developed from a reconstructed embryo, or is descended from a goat which developed from a reconstructed embryo, which was formed from genetic material from the same animal, an animal of the same genotype, or same cell line, which supplied the genetic material for the first goat. In a preferred embodiment, a first transgenic goat which develops from the reconstructed embryo can be mated with a second transgenic goat which developed from a reconstructed embryo and which contains a different transgene than the first transgenic goat.

In a preferred embodiment, the goat is a male goat. In other preferred embodiments, the goat is a female goat. A female goat can be induced to lactate and milk can be obtained from the goat.

In a preferred embodiment: a product, e.g., a protein, e.g., a recombinant protein, e.g., a human protein, is recovered from the goat; a product, e.g., a protein, e.g., a human protein, is recovered from the milk, urine, hair, blood, skin or meat of the goat.

In a preferred embodiment, the caprine genome of the somatic cell includes a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest such as a protein, a polypeptide, or a peptide. A protein can be any of: a hormone, an immunoglobulin, a plasma protein, and an enzyme.. The transgenic sequence can encode any protein whose expression in the transgenic goat is desired including, but not limited to any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythtpoietin, tissue plasminogen activator, human growth factor, antithrombin III, insulin, prolactin, and α1-antitrypsin.

In a preferred embodiment, the transgenic sequence encodes a human protein.

In a preferred embodiment, the caprine genome comprises a heterologous transgenic sequence under the control of a promoter, e.g., a caprine promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be any of: a casein promoter, a beta lactoglobulin promoter, a whey acid protein promoter and a lactalbumin promoter.

The invention also includes a non-human animal made by any of the methods described herein. The methods described for goats can be applied for any non-human mammal. Accordingly, in another aspect, the invention features a cloned goat, or descendant thereof, obtained by introducing a caprine genome of a caprine somatic cell into a caprine oocyte, preferably a naturally matured telophase oocyte, to a obtain reconstructed embryo and allowing the reconstructed embryo to develop into a goat.

In a preferred embodiment, the caprine genome can be from an embryonic somatic cell. A somatic cell can be any of: fibroblast (e.g., a primary fibroblast), a muscle cell (e.g., a myocyte), a cumulus cell or a mammary cell. In a preferred embodiment, the somatic cell is a non-quiescent cell (e.g. the cell is activated), e.g., the somatic cell is in G₁ stage, e.g., in G₁ prior to START. In another preferred embodiment, the somatic cell is a quiescent cell (e.g., the cell is arrested), e.g., the somatic cell is in G₀ stage.

In a preferred embodiment, the caprine oocyte can be a functionally enucleated oocyte, e.g., an enucleated oocyte.

In a preferred embodiment, the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is activated prior to or simultaneously with the introduction of the genome. In a preferred embodiment, the oocyte and somatic cell are synchronized, e.g., both the oocyte and somatic cell are activated or both the oocyte and somatic cell are arrested.

In a preferred embodiment, the caprine genome can be introduced by fusing, e.g., by electrofusion, of a somatic cell with the functionally enucleated oocyte.

In another aspect, the invention features one, or more, e.g., a population having at least one male and one female, cloned goat, each cell of which has its chromosomal genome derived from a caprine somatic cell, wherein said caprine somatic cell is from a goat other than cloned goat.

In a preferred embodiment, the chromosomal genome can be from an embryonic somatic cell. A somatic cell can be any of: a fibroblast (e.g., a primary fibroblast), a muscle cell (e.g., a myocyte), a neural cell, a cumulus cell or a mammary cell. In a preferred embodiment, the somatic cell is a non-quiescent cell (e.g., the cell is activated), e.g., the somatic cell is in G₁ stage, e.g., in G₁ prior to START. In another preferred embodiment, the somatic cell is a quiescent cell (e.g., the cell is arrested), e.g., the somatic cell is in G₀ stage.

In another aspect, the invention features a transgenic goat, or descendant thereof, obtained by introducing a caprine genome of a genetically engineered caprine somatic cell into a caprine oocyte, preferably a naturally matured telophase oocyte, to obtain a reconstructed embryo and allowing the reconstructed embryo to develop into a goat.

In a preferred embodiment, the caprine genome can be from an embryonic somatic cell. In another preferred embodiment, the caprine genome can be from a caprine fibroblast, e.g., an embryonic fibroblast.

In a preferred embodiment, the caprine oocyte can be a functionally enucleated oocyte, e.g., an enucleated oocyte.

In a preferred embodiment, the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is activated prior to or simultaneously with the introduction of the genome. In a preferred embodiment, the oocyte and the somatic cell are synchronized, e.g., both the oocyte and the somatic cell are activated or both the oocyte and the somatic cell are arrested.

In a preferred embodiment, the caprine genome can be introduced by fusing, e.g., by electrofusion, of a somatic cell with the functionally enucleated oocyte.

In a preferred embodiment, the caprine genome of the somatic cell includes a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode a protein which can be any of: a hormone, an immunoglobulin, a plasma protein, an enzyme, and a peptide. The transgenic sequence can encode any product of interest such as a protein, a polypeptide or a peptide. A protein which can be any protein whose expression in the transgenic goat is desired including, but not limited to any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the caprine genome comprises a heterologous transgenic sequence under the control of a promoter, e.g., a caprine promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be any of: a casein promoter, a beta lactoglobulin promoter, a whey acid protein promoter and a lactalbumin promoter.

In another aspect, the invention features a transgenic goat, each cell of which has its chromosomal genome derived from a genetically engineered caprine somatic cell, wherein said caprine somatic cell is from a goat other than said transgenic goat.

In a preferred embodiment, the chromosomal genome can be from an embryonic somatic cell. In another preferred embodiment, the chromosomal genome can be from a caprine fibroblast, e.g., an embryonic fibroblast.

In a preferred embodiment, the chromosomal genome of the somatic cell includes a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest such as a protein, a polypeptide and a peptide. A protein can be any of: a hormone, an immunoglobulin, a plasma protein, an enzyme, and a peptide. The transgenic sequence can encode any protein whose expression in the transgenic goat is desired including, but not limited to any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the chromosomal genome comprises a heterologous transgenic sequence under the control of a promoter, e.g., a caprine promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be any of: a casein promoter, a beta lactoglobulin promoter, a whey acid protein promoter and a lactalbumin promoter.

In another aspect, the invention features a goat made by mating a goat which developed from a reconstructed embryo (made as described herein) with a second goat.

In a preferred embodiment: the second goat developed from a reconstructed embryo or is descended from a goat which developed from a reconstructed embryo; the second goat developed from a reconstructed embryo, or is descended from a goat which developed from a reconstructed embryo, which was formed from genetic material from the same animal, an animal of the same genotype, or same cell line, which supplied the genetic material for the first goat. In a preferred embodiment, a first transgenic goat which develops from the reconstructed embryo can be mated with a second transgenic goat which developed from a reconstructed embryo and which contains a different transgene than the first transgenic goat.

In another aspect, the invention features a plurality of transgenic goats obtained by mating a goat which developed from a reconstructed embryo with a second goat.

In a preferred embodiment: the second goat developed from a reconstructed embryo or is descended from a goat which developed from a reconstructed embryo; the second goat developed from a reconstructed embryo, or is descended from a goat which developed from a reconstructed embryo, which was formed from genetic material from the same animal, an animal of the same genotype, or same cell line, which supplied the genetic material for the first goat. In a preferred embodiment, a first goat which developed from a reconstructed embryo can be mated with a second goat which developed from a reconstructed embryo and which contains a different transgene than the first goat.

In yet another aspect, the invention features a method of providing a transgenic goat which is homozygous for a transgenic sequence. The method includes providing a somatic cell which is heterozygous for a transgenic sequence; allowing somatic recombination to occur so as to produce a somatic cell which is homozygous for the transgenic sequence; introducing the genome from the somatic cell which is homozygous for the transgenic sequence into a caprine oocyte, preferably a naturally matured telophase oocyte, to form a reconstructed embryo; and allowing the reconstructed embryo to develop into a goat, e.g., by introducing the reconstructed embryo into a recipient doe, thereby providing a transgenic goat which is homozygous for a transgenic sequence.

In another aspect, the invention features a transgenic goat which is homozygous for a transgenic sequence.

In a preferred embodiment, the transgenic goat was made by introducing the genome from the somatic cell which is homozygous for the transgenic sequence into a caprine oocyte, preferably a naturally matured telophase oocyte, to form a reconstructed embryo; and allowing the reconstructed embryo to develop into a goat.

In another aspect, the invention features a method of making a cloned non-human mammal, e.g., a goat, cow, pig, horse, sheep, llama, camel. The method includes providing an activated oocyte, e.g., an oocyte in telophase stage, preferably a naturally matured telophase oocyte; functionally enucleating the oocyte; introducing the chromosomal genome of a somatic cell into the functionally enucleated oocyte to obtain a reconstructed embryo; and allowing the reconstructed embryo to develop ,e.g., by introducing the reconstructed embryo into a recipient doe, thereby making a cloned mammal.

In a preferred embodiment, the mammal, e.g., a goat, develops from the reconstructed embryo. In another embodiment, the mammal, e.g., a goat, is a descendant of a mammal, e.g., a goat which developed from the reconstructed embryo.

In a preferred embodiment, the somatic cell is an embryonic somatic cell. In a preferred embodiment the somatic cell is a fibroblast, e.g., an embryonic fibroblast. In a preferred embodiment, the somatic cell is a non-quiescent cell (e.g., the cell is activated), e.g., the somatic cell is in G₁ stage, e.g., in G₁ prior to START. In another preferred embodiment, the somatic cell is a quiescent cell (e.g., the cell is arrested), e.g., the somatic cell is in G₀ stage.

In a preferred embodiment, the oocyte is activated prior to or simultaneously with the introduction of the genome. In a preferred embodiment, the oocyte is obtained using an *in vivo* protocol, e.g., the oocyte is obtained using an *in vivo* protocol to obtain an oocyte which is in a desired stage of the cell cycle, e.g., metaphase II or telophase. In a preferred embodiment, the oocyte and somatic cell are synchronized, e.g., both the oocyte and somatic cell are activated or both the oocyte and somatic cell are arrested.

In a preferred embodiment, the chromosomal genome of the somatic cell is introduced into the oocyte by fusion, e.g., electrofusion, or by direct injection of the nucleus into the oocyte, e.g., microinjection.

In another aspect, the invention features a cloned non-human mammal, e.g., a goat, cow, pig, horse, sheep, llama, camel, obtained by functionally enucleating an activated oocyte, e.g., an oocyte in telophase, and introducing the chromosomal genome of a somatic cell into the enucleated oocyte, preferably a naturally matured telophase oocyte, to form a reconstructed embryo; and allowing the reconstructed embryo to develop, e.g., by introducing the reconstructed embryo into a recipient mammal.

In a preferred embodiment, the oocyte is obtained using an *in vivo* protocol, e.g., the oocyte is obtained using an *in vivo* protocol to obtain an oocyte which is in a desired stage of the cell cycle, e.g., telophase.

In another aspect, the invention features a reconstructed non-human mammalian embryo, e.g., a goat, cow, pig, horse, sheep, llama, camel embryo, obtained by functionally enucleating an activated oocyte, e.g., an oocyte in telophase, preferably a naturally matured telophase oocyte, and introducing the chromosomal genome of a somatic cell into the enucleated oocyte.

In a preferred embodiment, the oocyte is obtained using an *in vivo* protocol; the oocyte is obtained using an *in vivo* protocol to obtain an oocyte which is in a desired stage of the cell cycle, e.g., telophase

In yet another aspect, the invention features a method of making a transgenic non-human mammal, e.g., a goat, cow, pig, horse, sheep, llama, camel. The method includes providing an activated oocyte, e.g., an oocyte intelophase stage, preferably a naturally matured telophase oocyte; functionally enucleating the oocyte; introducing the chromosomal genome of a genetically engineered somatic cell into the functionally enucleated oocyte to obtain a reconstructed embryo; and allowing the reconstructed embryo to develop, e.g., by introducing the reconstructed embryo into a recipient female, such that a transgenic mammal is obtained.

In a preferred embodiment, the mammal develops from the reconstructed embryo. In another embodiment, the mammal is a descendant of a mammal which developed from the reconstructed embryo.

In a preferred embodiment, the somatic cell is an embryonic somatic cell. In another preferred embodiment, the somatic cell is a fibroblast, e.g., an embryonic fibroblast. In a preferred embodiment, the somatic cell is a non-quiescent cell (e.g., the cell is activated), e.g., the somatic cell is in G₁ stage, e.g., in G₁ prior to START. In another preferred embodiment, the somatic cell is a quiescent cell (e.g., the cell is arrested), e.g., the somatic cell is in G₀ stage.

In a preferred embodiment, the oocyte is activated prior to or simultaneously with the introduction of the genome. In a preferred embodiment, the oocyte is obtained using an *in vivo* protocol, e.g., the oocyte is obtained using an *in vivo* protocol to obtain an oocyte which is in a desired stage of the cell cycle, e.g., telophase. In a preferred embodiment, the oocyte and somatic cell are synchronized, e.g., both the oocyte and somatic cell are activated or both the oocyte and somatic cell are arrested. In a preferred embodiment, the chromosomal genome of the somatic cell is introduced into the oocyte by fusion, e.g., electrofusion, or by direct injection of the nucleus into the oocyte, e.g., microinjection.

In a preferred embodiment, the nucleus of the somatic cell comprises a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest such as a protein, a polypeptide and a peptide. A protein can be any of: a hormone, an immunoglobulin, a plasma protein, and an enzyme. The transgenic sequence can encode any protein whose expression in the transgenic mammal is desired including, but not limited to any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the chromosomal genome comprises a heterologous transgenic sequence under the control of a promoter, e.g., a mammalian-specific promoter, e.g., a caprine promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be any of: a casein promoter, a beta lactoglobulin promoter, a whey acid protein promoter and a lactalbumin promoter.

In another aspect, the invention features a transgenic non-human mammal, e.g., a goat, cow, pig, horse, sheep, llama, camel, made by functionally enucleating an activated oocyte, e.g., an oocyte in telophase, preferably a naturally matured telophase oocyte, and introducing the chromosomal genome of a genetically engineered somatic cell into the enucleated oocyte to form a reconstructed embryo and allowing the reconstructed embryo to develop, e.g., by introducing the reconstructed embryo into a recipient mammal.

In a preferred embodiment, the oocyte is obtained using an *in vivo* protocol, e.g., the oocyte is obtained using an *in vivo* protocol to obtain an oocyte which is in a desired stage of the cell cycle, e.g., telophase.

In another aspect, the invention features a reconstructed non-human mammalian embryo, e.g., a goat, cow, pig, horse, sheep, llama, camel embryo, obtained by functionally enucleating an activated oocyte, e.g., an oocyte in telophase, preferably a naturally matured telophase oocyte, and introducing the chromosomal genome of a genetically engineered somatic cell into the enucleated oocyte.

In a preferred embodiment, the oocyte is obtained using an *in vivo* protocol, e.g., the oocyte is obtained using an *in vivo* protocol to obtain an oocyte which is in a desired stage of the cell cycle, e.g., telophase.

In yet another aspect, the invention features a method of making a cloned non-human mammal, e.g., a goat, cow, pig, horse, sheep, llama, camel. The method includes providing an oocyte, preferably a naturally matured telophase oocyte; functionally enucleating the oocyte; introducing the chromosomal genome of a somatic cell into the functionally enucleated oocyte to obtain a reconstructed embryo, wherein the oocyte is activated prior to or simultaneously with the introduction of the chromosomal genome; introducing the reconstructed embryo into a recipient mammal; and allowing the reconstructed embryo to develop, thereby making a cloned mammal.

In a preferred embodiment, the mammal develops from the reconstructed embryo. In another embodiment, the mammal is a descendant of a mammal which developed from the reconstructed embryo.

In a preferred embodiment, the somatic cell is an embryonic cell. In another preferred embodiment, the somatic cell is a fibroblast, e.g., an embryonic fibroblast. In a preferred embodiment, the somatic cell is a non-quiescent cell (e.g., the cell is activated), e.g., the somatic cell is in G₁ stage, e.g., in G₁ prior to START. In another preferred embodiment, the somatic cell is a quiescent cell (e.g., the cell is arrested), e.g., the somatic cell is in G₀ stage. In another preferred embodiment, the oocyte is an enucleated oocyte.

In a preferred embodiment, the oocyte is in metaphase II; the oocyte is in telophase; the oocyte is obtained using an *in vivo* protocol, e.g., the oocyte is obtained using an *in vivo* protocol to obtain an oocyte which is in a desired stage of the cell cycle, e.g., telophase. In a preferred embodiment, the oocyte and somatic cell are synchronized, e.g., both the oocyte and somatic cell are activated or both the oocyte and somatic cell are arrested.

In a preferred embodiment, the chromosomal genome of the somatic cell is introduced into the oocyte by fusion, e.g., electrofusion, or by direct injection of the nucleus into the oocyte, e.g., microinjection.

In another aspect, the invention features a cloned non-human mammal, e.g., a goat, cow, pig, horse, sheep, llama, camel, made by functionally enucleating a mammalian oocyte, preferably a naturally matured telophase oocyte, and activating the oocyte prior to or simultaneously with the introduction of the chromosomal genome of a somatic cell into the enucleated oocyte.

In yet another aspect, the invention features a reconstructed non-human mammalian embryo, e.g., a goat, cow, pig, horse, sheep, llama, camel embryo, obtained by functionally enucleating a mammalian oocyte, preferably a naturally matured telophase oocyte, and activating the oocyte prior to and/or simultaneously with the introduction of the chromosomal genome of a somatic cell into the enucleated oocyte.

In another aspect, the invention features a method of making a transgenic non-human mammal, e.g., a goat, cow, pig, horse, sheep, llama, camel. The method includes providing an oocyte, preferably a naturally matured telophase oocyte; functionally enucleating the oocyte; introducing the chromosomal genome of a genetically engineered somatic cell into the functionally enucleated oocyte to obtain a reconstructed embryo, wherein the oocyte is activated prior to or simultaneously with the introduction ofthe chromosomal genome; and allowing the reconstructed embryo to develop, e.g., by introducing the reconstructed embryo into a recipient mammal, such that a transgenic mammal is obtained.

In a preferred embodiment, the mammal develops from the reconstructed embryo. In another embodiment, the mammal is a descendant of a mammal which developed from the reconstructed embryo.

In a preferred embodiment, the somatic cell is an embryonic somatic cell. In another preferred embodiment, the somatic cell is a fibroblast, e.g., an embryonic fibroblast. In a preferred embodiment, the somatic cell is a non-quiescent cell (e.g., the cell is activated), e.g., the somatic cell is in G₁ stage, e.g., in G₁ prior to START. In another preferred embodiment, the somatic cell is a quiescent cell (e.g., the cell is arrested), e.g., the somatic cell is in G₀ stage. In another preferred embodiment, the oocyte is an enucleated oocyte

In a preferred embodiment, the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is activated prior to or simultaneously with the introduction of the genome. In a preferred embodiment, the oocyte and somatic cell are synchronized, e.g., both the oocyte and the somatic cell are activated or both the oocyte and somatic cell are arrested.

In a preferred embodiment, the chromosomal genome of the somatic cell is introduced into the oocyte by fusion, e.g., electrofusion, or by direct injection of the nucleus into the oocyte, e.g., microinjection.

In a preferred embodiment, the nucleus of the somatic cell comprises a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a tissue-specific promoter. The transgenic sequence can encode any product of interest such as a protein, a polypeptide and a peptide. A protein can be any of: a hormone, an immunoglobulin, a plasma protein, and an enzyme. The transgenic sequence can encode a protein whose expression in the transgenic mammal is desired including, but not limited to any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the chromosomal genome comprises a heterologous transgenic sequence under the control of a promoter, e.g., a mammalian-specific promoter, e.g., a caprine promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be any of: a casein promoter, a beta lactoglobulin promoter, a whey acid protein promoter and a lactalbumin promoter.

In another aspect, the invention features a transgenic non-human mammal, e.g., a goat, cow, pig, horse, sheep, llama, camel, made by functionally enucleating a mammalian oocyte, preferably a naturally matured telophase oocyte, and activating the oocyte prior to or simultaneously with the introduction of the chromosomal genome of a genetically engineered somatic cell into the enucleated oocyte.

In yet another aspect, the invention features a reconstructed non-human mammalian embryo, e.g., a goat, cow, pig, horse, sheep, llama, camel embryo, obtained by functionally enucleating a mammalian oocyte, preferably a naturally matured telophase oocyte, and activating the oocyte prior to or simultaneously with the introduction of the chromosomal genome of a genetically engineered somatic cell into the enucleated oocyte.

The invention also includes a product, e.g., a protein, e.g., a heterologous protein, described herein obtained from a non-human mammal, e.g., a cloned or transgenic mammal, e.g., a cloned or transgenic goat, described herein.

In a preferred embodiment, product is milk or a protein secreted into milk.

In another aspect, the invention features a method of providing a protein, e.g., a human protein. The method includes: providing a non-human mammal, e.g., a transgenic mammal, e.g., a transgenic goat, described herein; and recovering the product from the mammal, or from a product, e.g., milk, of the mammal.

In another aspect, the invention features a method of providing a heterologous polypeptide. The methods includes introducing a caprine genome, e.g., by introducing a nucleus, of a genetically engineered caprine somatic cell into a caprine oocyte, preferably a naturally matured telophase oocyte, to form a reconstructed embryo; allowing the reconstructed embryo to develop into a goat, e.g., by introducing the reconstructed embryo into a recipient doe; and recovering the polypeptide from the goat or a descendant thereof.

In a preferred embodiment, the nucleus of the caprine somatic cell is introduced into the caprine oocyte, e.g., by direct nuclear injection or by fusion, e.g., electrofusion, of the somatic cell with the oocyte.

In a preferred embodiment: the somatic cell is a non-quiescent cell (e.g., the cell is activated), e.g., the somatic cell is in G₁ stage, e.g., in G₁ prior to START. In another preferred embodiment, the somatic cell is a quiescent cell (e.g., the cell is arrested), e.g., the somatic cell is in G₀ stage. In a preferred embodiment, the somatic cell is an embryonic somatic cell, e.g., an embryonic fibroblast. The somatic cell can be a fibroblast (e.g., a primary fibroblast), a muscle cell (e.g., a myocyte), a neural cell, a cumulus cell or a mammary cell.

In a preferred embodiment, a transgenic sequence has been introduced into the somatic cell; the somatic cell is from a cell line, e.g., a primary cell line; the somatic cell is from a cell line and a transgenic sequence has been inserted into the cell.

In a preferred embodiment, the oocyte is a functionally enucleated oocyte, e.g., an enucleated oocyte.

In a preferred embodiment, the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is activated prior to or simultaneously with the introduction of the genome. In a preferred embodiment, the oocyte and the somatic cell are synchronized, e.g., both the oocyte and the somatic cell are activated or both the oocyte and the somatic cell are arrested.

In a preferred embodiment, the caprine genome of the somatic cell includes a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest including a protein, a polypeptide and a peptide. A protein can be any of: a hormone, an immunoglobulin, a plasma protein, and an enzyme. The transgenic sequence can encode any protein whose expression in the transgenic goat is desired including, but not limited to any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the caprine genome comprises a heterologous transgenic sequence under the control of a promoter, e.g., a caprine promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be any of: a casein promoter, a beta lactoglobulin promoter, a whey acid protein promoter and a lactalbumin promoter.

In another aspect, the invention features a method of making a heterologous polypeptide. The method includes fusing a genetically engineered caprine somatic cell which comprises a transgene encoding a heterologous polypeptide and a milk-specific promoter, with an enucleated caprine oocyte, preferably a naturally matured telophase oocyte, to obtain a reconstructed embryo; and allowing the reconstructed embryo to develop into a transgenic goat, e.g., by introducing the reconstructed embryo into a recipient doe.

In a preferred embodiment, the transgene is operatively linked to the milk-specific promoter. The milk-specific promoter can be any of: a casein promoter, a beta lactoglobulin promoter, a whey acid protein promoter and a lactalbumin promoter.

In a preferred embodiment, the nucleus of the caprine somatic cell is introduced into the caprine oocyte, e.g., by direct nuclear injection or by fusion, e.g., electrofusion, of the somatic cell with the oocyte.

In a preferred embodiment, the somatic cell is an embryonic somatic cell. In another preferred embodiment, the somatic cell is a fibroblast, e.g., an embryonic fibroblast.

In a preferred embodiment, the oocyte is a functionally enucleated oocyte, e.g., an enucleated oocyte.

In a preferred embodiment, the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is activated prior to or simultaneously with the introduction of the genome. In a preferred embodiment, the oocyte and the somatic cell are synchronized, e.g., both the oocyte and the somatic cell are activated or both the oocyte and the somatic cell are arrested.

In a preferred embodiment, the caprine genome of the somatic cell includes a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest such as a protein, a polypeptide or a peptide. A protein can be any of: a hormone, an immunoglobulin, a plasma protein, an enzyme. The transgenic sequence can encode a protein whose expression in the transgenic goat is desired including, but not limited to any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the heterologous polypeptide is purified from the milk of the transgenic goat.

In a preferred embodiment, the method can also include milking the transgenic goat.

In another aspect, the invention features a method of providing a heterologous polypeptide. The method includes obtaining a goat made by introducing a caprine genome of a genetically engineered caprine somatic cell into a caprine oocyte, preferably a naturally matured telophase oocyte, to form a reconstructed embryo; and allowing the reconstructed embryo to develop into a goat, e.g., by introducing the reconstructed embryo into a recipient doe; and recovering the polypeptide from the goat, e.g., from the milk of the goat, or a descendant thereof.

In a preferred embodiment, the caprine genome of the somatic cell includes a transgenic sequence. The transgenic sequence can be any of: integrated into the genome; a heterologous transgene, e.g., a human transgene; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest such as a protein, a polypeptide and a peptide. A protein can be any of: a hormone, an immunoglobulin, a plasma protein, and an enzyme. The transgenic sequence can encode any protein whose expression in the transgenic goat is desired including, but not limited to any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the heterologous polypeptide is purified from the milk of the transgenic goat.

In another aspect, the invention features method of making a reconstructed caprine embryo. The method includes introducing a caprine genome from a caprine somatic cell into a caprine oocyte, preferably a naturally matured telophase oocyte, thereby forming a reconstructed embryo.

In a preferred embodiment: the somatic cell is a non-quiescent cell (e.g., the cell is activated), e.g., the somatic cell is in G₁ stage. In another preferred embodiment, the somatic cell is a quiescent cell (e.g., the cell is arrested), e.g., the somatic cell is in G₀ stage. In a preferred embodiment, the somatic cell is an embryonic somatic cell, e.g., an embryonic fibroblast. The somatic cell can be a fibroblast (e.g., a primary fibroblast), a muscle cell (e.g., a myocyte), a neural cell, a cumulus cell or a mammary cell.

In a preferred embodiment: the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is enucleated. In a preferred embodiment, the oocyte and the somatic cell are synchronized, e.g., both the oocyte and the somatic cell are activated or both the oocyte and the somatic cell are arrested.

In yet another aspect, the invention features a reconstructed caprine embryo obtained by introducing a caprine genome from a caprine somatic cell into a caprine oocyte, preferably a naturally matured telophase oocyte.

In another aspect, the invention features a method of making a reconstructed transgenic caprine embryo. The method includes introducing a caprine genome, e.g., by introducing a nucleus, of a genetically engineered caprine somatic cell into a caprine oocyte, preferably a naturally matured telophase oocyte, thereby forming a transgenic reconstructed embryo.

In a preferred embodiment the somatic cell is a non-quiescent cell (i.e., the cell is activated), e.g., the somatic cell is in G₁ stage. In another preferred embodiment, the somatic cell is a quiescent cell (i.e., the cell is arrested), e.g., the somatic cell is in G₀ stage. In a preferred embodiment, the somatic cell is an embryonic somatic cell, e.g., an embryonic fibroblast. The somatic cell can be a fibroblast (e.g., a primary fibroblast), a muscle cell (e.g., a myocyte), a neural cell, a cumulus cell or a mammary cell.

In a preferred embodiment: the oocyte is in metaphase II; the oocyte is in telophase; the oocyte is obtained using an *in vivo* protocol; the oocyte is obtained using an *in vivo* protocol to obtain an oocyte which is in a desired stage of the cell cycle, e.g., metaphase II or telophase; the oocyte is enucleated. In a preferred embodiment, the oocyte and the somatic cell are synchronized, e.g., both the oocyte and the somatic cell are activated or both the oocyte and somatic cell are arrested.

In another aspect, the invention features a reconstructed transgenic caprine embryo obtained by introducing a caprine genome, e.g., by introducing a nucleus, of a genetically engineered caprine somatic cell into a caprine oocyte, preferably a naturally matured telophase oocyte.

In another aspect, the invention features a method of providing a herd of goats. The method includes making a first goat by introducing a caprine genome, e.g., by introducing the nucleus, from a caprine somatic cell into a caprine oocyte, preferably a naturally matured telophase oocyte, to form a reconstructed embryo and allowing the reconstructed embryo to develop into the first goat; making a second goat by introducing a caprine genome, e.g., by introducing the nucleus, from a caprine somatic cell into a caprine oocyte, preferably a naturally matured telophase oocyte, to form a reconstructed embryo and allowing the reconstructed embryo to develop into the second goat; whereby the genome of the first and second goats are from the genetic material of the same animal, same genotype or same cell line, thereby providing a herd of goats.

In a preferred embodiment, the first goat, or descendant thereof, is mated with the second goat or a descendant thereof.

In another aspect, the invention features a herd of goats obtained by making a first goat by introducing a caprine genome, e.g., by introducing the nucleus, from a caprine somatic cell into a caprine oocyte, preferably a naturally matured telophase oocyte, to form a reconstructed embryo and allowing the reconstructed embryo to develop into the first goat; making a second goat by introducing a caprine genome, e.g., by introducing the nucleus, from a caprine somatic cell into a caprine oocyte to form a reconstructed embryo and allowing the reconstructed embryo to develop into the second goat; whereby the genome of the first and second goats are from the genetic material of the same animal, same genotype or same cell line.

In a preferred embodiment, the herd of goats is obtained by any of the methods described herein.

In another aspect, the invention features, an embryonic or fetal caprine somatic cell.

In a preferred embodiment, the cell is a purified embryonic or fetal caprine somatic cell.

In a preferred embodiment, the cell is in a preparation of embryonic or fetal caprine somatic cells.

In a preferred embodiment, the cell can be used to derive an embryonic or fetal caprine somatic cell line.

In a preferred embodiment, the cell includes a transgene, e.g., a transgene encoding a polypeptide. The transgene can be: integrated into the genome of the somatic cell; a heterologous transgene, e.g., a heterologous transgene which includes a human sequence; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode a product of interest such as a protein, polypeptide or peptide.

In a preferred embodiment, the transgene encodes any of: a hormone, an immunoglobulin, a plasma protein, and an enzyme. The transgene can encode, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin:

In a preferred embodiment, the transgene is under the control of a promoter, e.g., a heterologous or a caprine promoter. The promoter can be a tissue-specific promoter. The tissue specific promoter can be any of: a milk-specific promoter, a blood-specific promoter; a muscle-specific promoter; a neural-specific promoter; a skin-specific promoter; a hair specific promoter; and, a urine-specific promoter. The milk-specific promoter can be, e.g., any of: a β-casein promoter; a β-lactoglobin promoter; a whey acid protein promoter; and a lactalbumin promoter.

In a preferred embodiment, the somatic cell is a fibroblast. The fibroblast can be a primary fibroblast or a primary derived fibroblast.

In a preferred embodiment, the cell is obtained from a goat, e.g., an embryonic goat, derived from a germ cell obtained from a transgenic mammal. The germ cell can be sperm from a transgenic goat.

In a preferred embodiment, the cell is a genetically engineered embryonic or fetal caprine somatic cell, e.g., a purified genetically engineered embryonic or fetal caprine somatic cell.

In a preferred embodiment, the cell is part of a preparation of genetically engineered embryonic or fetal caprine somatic cells. In another preferred embodiment, the cell is used to derive a genetically engineered embryonic or fetal caprine somatic cell line.

In a preferred embodiment, the genetically engineered cell includes a nucleic acid, e.g., a nucleic acid encoding a polypeptide, which has been introduced into the cell. The nucleic acid can be: integrated into the genome of the somatic cell; a heterologous nucleic acid, e.g., a heterologous nucleic acid which includes a human sequence; a knockout, knockin or other event which disrupts the expression of a caprine gene; a sequence which encodes a protein, e.g., a human protein; a heterologous promoter; a heterologous sequence under the control of a promoter, e.g., a caprine promoter. The nucleic acid sequence can encode any product of interest such as a protein, polypeptide or peptide.

In a preferred embodiment, the nucleic acid encodes any of: a hormone, an immunoglobulin, a plasma protein, and an enzyme. The nucleic acid can encode, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the nucleic acid is under the control of a promoter, e.g., a caprine or heterologous promoter. The promoter can be a tissue-specific promoter. The tissue specific promoter can be any of: a milk-specific promoter; a blood-specific promoter; a muscle-specific promoter, a neural-specific promoter; a skin-specific promoter; a hair specific promoter; and, a urine-specific promoter. The milk-specific promoter can be, e.g., any of: a β-casein promoter; a β-lactoglobin promoter; a whey acid protein promoter; and a lactalbumin promoter.

In a preferred embodiment, the somatic cell is a fibroblast. The fibroblast can be a primary fibroblast or a primary derived fibroblast.

In a preferred embodiment, the cell is obtained from a goat, e.g., an embryonic goat, derived from a germ cell obtained from a transgenic goat. The germ cell can be sperm or an oocyte from a transgenic goat.

In a preferred embodiment, the cell is used as a source of genetic material for nuclear transfer.

In another aspect, the invention features an embryonic or fetal caprine somatic cell, a preparation of cells, or an embryonic or fetal caprine somatic cell line, e.g., as described herein, in a container, e.g., an airtight or liquid tight container.

In another aspect, the invention features an embryonic or fetal caprine somatic cell, a preparation of cells, or an embryonic or fetal caprine somatic cell line, e.g., as described herein, which is frozen, e.g., is cryopreserved.

In another aspect, the invention features a kit. The kit includes a container of the cell or cells described herein. In a preferred embodiment, the kit further includes instructions for use in preparing a transgenic animal.

In a preferred embodiment, the kit further includes a recipient oocyte, e.g., an enucleated oocyte.

In another aspect, the invention features a method for providing a component for the production of a cloned or transgenic goat. The method includes obtaining a frozen sample of the cell or cells, e.g., those described herein, and thawing the sample.

In another aspect, the invention features, a method of preparing an embryonic or fetal caprine somatic cell line. The method includes obtaining a somatic cell from an embryonic or fetal goat; and, culturing the cell, e.g., in a suitable medium, such that a somatic cell line is obtained.

In a preferred embodiment, the cell line is a genetically engineered cell line, e.g., the cell comprises a transgene. The transgene can be: integrated into the somatic cell genome; a heterologous transgene, e.g., a heterologous transgene which includes a human sequence; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest such as a protein, polypeptide or peptide.

In a preferred embodiment, the transgene encodes any of: a hormone, an immunoglobulin, a plasma protein, and an enzyme. The transgene can encode any protein, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgene is under the control of a promoter, e.g., a caprine or heterologous promoter. The promoter can be a tissue-specific promoter. The tissue specific promoter can be any of: a milk-specific promoter; a blood-specific promoter, a muscle-specific promoter, a neural-specific promoter; a skin-specific promoter; a hair specific promoter; and, a urine-specific promoter. The milk-specific promoter can be, e.g., any of: a β-casein promoter; a β-lactoglobin promoter; a whey acid protein promoter; and a lactalbumin promoter.

In a preferred embodiment, the genetically engineered cell includes a nucleic acid, e.g., a nucleic acid encoding a polypeptide, which has been introduced into the cell. The nucleic acid can be: integrated into the genome of the somatic cell; a heterologous nucleic acid, e.g., a heterologous nucleic acid which includes a human sequence; a knockout, knockin or other event which disrupts the expression of a caprine gene; a sequence which encodes a protein, e.g., a human protein; a heterologous promoter; a heterologous sequence under the control of a promoter, e.g., a caprine promoter. The nucleic acid sequence can encode any product of interest such as a protein, polypeptide or peptide.

In a preferred embodiment, the nucleic acid can encode any of: a hormone, an immunoglobulin, a plasma protein, and an enzyme. The nucleic acid can encode, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the nucleic acid is under the control of a promoter, e.g., a caprine or heterologous promoter. The promoter can be a tissue-specific promoter. The tissue specific promoter can be any of: a milk-specific promoter; a blood-specific promoter; a muscle-specific promoter; a neural-specific promoter; a skin-specific promoter; a hair specific promoter; and, a urine-specific promoter. The milk-specific promoter can be, e.g., any of: a β-casein promoter; a β-lactoglobin promoter; a whey acid protein promoter; and a lactalbumin promoter.

In a preferred embodiment, the somatic cell is a fibroblast. The fibroblast can be a primary fibroblast or a primary derived fibroblast.

In a preferred embodiment, the cell is obtained from a goat, e.g., an embryonic or fetal goat, derived from a germ cell obtained from a transgenic goat. The germ cell can be sperm or an oocyte from a transgenic goat.

In a preferred embodiment, the cell is used as a source of genetic material for nuclear transfer.

In another aspect, the invention features, a method of preparing an embryonic or fetal caprine somatic cell line. The method includes inseminating a female recipient with the semen from a goat; obtaining a transgenic embryo from the recipient; obtaining a somatic cell from the embryo; and, culturing the cell in a suitable medium, such that a somatic cell line is obtained.

In a preferred embodiment, the semen is from a transgenic goat.

In a preferred embodiment, the cell line is a genetically engineered cell line, e.g., the cell comprises a transgene. The transgene can be: integrated into the somatic cell genome; a heterologous transgene, e.g., a heterologous transgene which includes a human sequence; a knockout, knockin or other event which disrupts the expression of a caprine gene; a sequence which encodes a protein, e.g., a human protein; a heterologous promoter; a heterologous sequence under the control of a promoter, e.g., a caprine promoter. The transgenic sequence can encode any product of interest such as a protein, polypeptide or peptide.

In a preferred embodiment, the transgene encodes any of: a hormone, an immunoglobulin, a plasma protein, and an enzyme. The transgene can encode any protein, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgene is under the control of a promoter, e.g., a caprine or heterologous promoter. The promoter can be a tissue-specific promoter. The tissue specific promoter can be any of: a milk-specific promoter, a blood-specific promoter; a muscle-specific promoter; a neural-specific promoter; a skin-specific promoter; a hair specific promoter; and, a urine-specific promoter. The milk-specific promoter can be, e.g., any of: a β-casein promoter; a β-lactoglobin promoter; a whey acid protein promoter; and a lactalbumin promoter.

In a preferred embodiment, the genetically engineered cell includes a nucleic acid, e.g., a nucleic acid encoding a polypeptide, which has been introduced into the cell. The nucleic acid can be: integrated into the genome of the somatic cell; a heterologous nucleic acid, e.g., a heterologous nucleic acid which includes a human sequence; a knockout, knockin or other event which disrupts the expression of a caprine gene; a sequence which encodes a protein, e.g., a human protein; a heterologous promoter; a heterologous sequence under the control of a promoter, e.g., a caprine promoter. The nucleic acid sequence can encode any product of interest such as a protein, polypeptide or peptide.

In a preferred embodiment, the nucleic acid can encodes any of: a hormone, an immunoglobulin, a plasma protein, and an enzyme. The nucleic acid can encode, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the nucleic acid is under the control of a promoter, e.g., a caprine or heterologous promoter. The promoter can be a tissue-specific promoter. The tissue specific promoter can be any of: a milk-specific promoter; a blood-specific promoter; a muscle-specific promoter, a neural-specific promoter; a skin-specific promoter; a hair specific promoter, and, a urine-specific promoter. The milk-specific promoter can be, e.g., any of: a β-casein promoter; a β-lactoglobin promoter; a whey acid protein promoter; and a lactalbumin promoter.

In a preferred embodiment, the somatic cell is a fibroblast. The fibroblast can be a primary fibroblast or a primary derived fibroblast.

In a preferred embodiment, the cell is used as a source of genetic material for nuclear transfer.

The present invention is also based, in part, on the discovery that a reconstructed embryo which is transferred into a recipient mammal at the two to four cell stage of embryogenesis can develop into a cloned mammal. The mammal can be an embryo, a fetus, or a post natal mammal, e.g., an adult mammal.

Accordingly, in one aspect, the invention features a method of producing a non-human mammal, e.g., a cloned mammal, e.g., a goat, cow, pig, horse, sheep, llama, camel. The method includes maintaining a mammalian reconstructed embryo, e.g., a reconstructed embryo wherein the genome is derived from a somatic cell, in culture until the embryo is in the 2 to 8 cell stage, transferring the embryo at the 2 to 8 cell stage into a recipient mammal, and allowing the reconstructed embryo to develop into a mammal, to thereby produce a mammal.

In a preferred embodiment, the mammal develops from the reconstructed embryo. In another embodiment, the mammal is a descendant of a mammal which developed from the reconstructed embryo.

In a preferred embodiment, the reconstructed embryo is maintained in culture until the embryo is in the 2 to 8, the 2 to 6, the 2 to 4 cell stage of embryogenesis.

In a preferred embodiment, the genome of the reconstructed embryo is derived from: a somatic cell, e.g., a fibroblast or epithelial cell; a genetically engineered somatic cell, e.g., a somatic cell comprising a transgenic sequence.

In a preferred embodiment, the method further includes mating the mammal which develops from the reconstructed embryo with: a second mammal; a second mammal which develops from a reconstructed embryo or is descended from a mammal which developed from a reconstructed embryo; or a second mammal developed from a reconstructed embryo, or descended from a mammal which developed from a reconstructed embryo, which was formed from genetic material from the same animal, an animal of the same genotype, or same cell line, which supplied the genetic material for the first mammal. In a preferred embodiment, a first transgenic mammal which develops from the reconstructed embryo can be mated with a second transgenic mammal which developed from a reconstructed embryo and which contains a different transgene that the first transgenic mammal.

In a preferred embodiment, the mammal is a male mammal. In other preferred embodiments, the mammal is a female mammal. A female mammal can be induced to lactate and milk can be obtained from the mammal.

In a preferred embodiment: a product, e.g., a protein, e.g., a recombinant protein, e.g., a human protein, is recovered from the mammal; a product, e.g., a protein, e.g., a human protein, is recovered from the milk, urine, hair, blood, skin or meat of the mammal.

In a preferred embodiment, the mammal is: embryonic; fetal; or, postnatal, e.g., adult.

In a preferred embodiment, the genome of the reconstructed embryo is derived from a genetically engineered somatic cell, e.g., a transgenic cell or a cell which a nucleic acid has been introduced.

In another aspect, the invention features a method of producing a non-human mammal, e.g., a transgenic mammal, e.g., a goat. cow, pig, horse, sheep, llama, camel. The method includes maintaining a mammalian reconstructed embryo (e.g., a reconstructed embryo wherein its genome is derived from a genetically engineered somatic cell) in culture until the embryo is in the 2 to 8 cell stage, transferring the embryo at the 2 to 8 cell stage into a recipient mammal, and allowing the reconstructed embryo to develop into a mammal, to thereby produce a transgenic mammal.

In a preferred embodiment, the mammal develops from the reconstructed embryo. In another embodiment, the mammal is a descendant of a mammal which developed from the reconstructed embryo.

In a preferred embodiment, the reconstructed embryo is maintained in culture until the embryo is in the 2 to 8, the 2 to 6, the 2 to 4 cell stage of embryogenesis.

In a preferred embodiment, the method further includes mating the mammal which develops from the reconstructed embryo with: a second mammal; a second mammal which develops from a reconstructed embryo or is descended from a mammal which developed from a reconstructed embryo; or a second mammal developed from a reconstructed embryo, or descended from a mammal which developed from a reconstructed embryo, which was formed from genetic material from the same animal, an animal of the same genotype, or same cell line, which supplied the genetic material for the first mammal. In a preferred embodiment, a first transgenic mammal which develops from the reconstructed embryo can be mated with a second transgenic mammal which developed from a reconstructed embryo and which contains a different transgene that the first transgenic mammal.

In a preferred embodiment, the mammal is a male mammal. In other preferred embodiments, the mammal is a female mammal. A female mammal can be induced to lactate and milk can be obtained from the mammal.

In a preferred embodiment: a product, e.g., a protein, e.g., a recombinant protein, e.g., a human protein, is recovered from the mammal; a product, e.g., a protein, e.g., a human protein, is recovered from the milk, urine, hair, blood, skin or meat of the mammal.

In a preferred embodiment, the genome of the genetically engineered somatic cell includes a transgenic sequence. The transgenic sequence can be any of: a heterologous transgene, e.g., a human transgerie; a knockout, knockin or other event which disrupts the expression of a mammalian gene; a sequence which encodes a protein, e.g., a human protein; a heterologous promoter; a heterologous sequence under the control of a promoter, e.g., a caprine promoter. The transgenic sequence can encode any product of interest such as a protein, polypeptide or peptide.

In a preferred embodiment, the transgenic sequence encodes any of: a hormone, an immunoglobulin, a plasma protein, and an enzyme. The transgenic sequence can encode any protein whose expression in the transgenic mammal is desired, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the transgenic sequence encodes a human protein.

In a preferred embodiment, the transgenic sequence is under the control of a promoter, e.g., a caprine or heterologous promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be, e.g., any of: a casein promoter, a beta lactoglobulin promoter, a whey acid protein promoter and a lactalbumin promoter.

In a preferred embodiment, a nucleic acid can be introduced into the genome of the genetically engineered somatic cell. The nucleic acid can be any of: a heterologous transgene, e.g., a human transgene; a knockout, knockin or other event which disrupts the expression of a mammalian gene; a sequence which encodes a protein, e.g., a human protein; a heterologous promoter, a heterologous sequence under the control of a promoter, e.g., a caprine or heterologous promoter. The nucleic acid sequence can encode any product of interest such as a protein, polypeptide or peptide.

In a preferred embodiment, the nucleic acid encodes any of: a hormone, an immunoglobulin, a plasma protein, and an enzyme. The nucleic acid sequence can encode any protein whose expression in the transgenic mammal is desired, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the nucleic acid sequence encodes a human protein.

In a preferred embodiment, the nucleic acid sequence is under the control of a promoter, e.g., a caprine or heterologous promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be, e.g., any of: a casein promoter, a beta lactoglobulin promoter, a whey acid protein promoter and a lactalbumin promoter.

In another aspect, the invention features a method of producing a cloned goat. The method includes maintaining a caprine reconstructed embryo (e.g., a reconstructed embryo wherein its genome is derived from a caprine somatic cell) in culture until the embryo is in the 2 to 8 cell stage, transferring the embryo at the 2 to 8 cell stage into a recipient goat, and allowing the reconstructed embryo to develop into a goat, to thereby produce a goat.

In a preferred embodiment, the goat is: embryonic; fetal; or, postnatal, e.g., adult

In a preferred embodiment, the goat develops from the reconstructed embryo. In another embodiment, the goat is a descendant of a goat which developed from the reconstructed embryo.

In a preferred embodiment, the reconstructed embryo is maintained in culture until the embryo is in the 2 to 8, the 2 to 6, the 2 to 4 cell stage of embryogenesis.

In a preferred embodiment, the genome of the reconstructed embryo is derived from: a caprine somatic cell, e.g., a fibroblast or epithelial cell; a genetically engineered caprine somatic cell, e.g., the genome of the caprine somatic cell comprises a transgenic sequence or a nucleic acid has been introduced into the genome of the somatic cell.

In a preferred embodiment, the method further includes mating the goat which develops from the reconstructed embryo with: a second goat; a second goat which develops from a reconstructed embryo or is descended from a goat which developed from a reconstructed embryo; or a second goat developed from a reconstructed embryo, or descended from a goat which developed from a reconstructed embryo, which was formed from genetic material from the same animal, an animal of the same genotype, or same cell line, which supplied the genetic material for the first goat. In a preferred embodiment, a first transgenic goat which develops from the reconstructed embryo can be mated with a second transgenic goat which developed from a reconstructed embryo and which contains a different transgene that the first transgenic goat.

In a preferred embodiment, the goat is a male goat. In other preferred embodiments, the goat is a female goat. A female goat can be induced to lactate and milk can be obtained from the goat.

In a preferred embodiment: a product, e.g., a protein, e.g., a recombinant protein, e.g., a human protein, is recovered from the goat; a product, e.g., a protein, e.g., a human protein, is recovered from the milk, urine, hair, blood, skin or meat of the goat.

In another aspect, the invention features a method of producing a transgenic goat. The method includes maintaining a caprine reconstructed embryo (e.g., a reconstructed embryo wherein its genome is derived from a genetically engineered somatic cell) in culture until the embryo is in the 2 to 8 cell stage, transferring the embryo at the 2 to 8 cell stage into a recipient goat, and allowing the reconstructed embryo to develop into a goat, to thereby produce a transgenic goat.

In a preferred embodiment, the goat is: embryonic; fetal; or, postnatal, e.g., adult.

In a preferred embodiment, the goat develops from the reconstructed embryo. In another embodiment, the goat is a descendant of a goat which developed from the reconstructed embryo.

In a preferred embodiment, the reconstructed embryo is maintained in culture until the embryo is in the 2 to 8, the 2 to 6, the 2 to 4 cell stage of embryogenesis.

In a preferred embodiment, the genome of the reconstructed embryo is derived from a somatic cell, e.g., a fibroblast or epithelial cell.

In a preferred embodiment, the method further includes mating the goat which develops from the reconstructed embryo with: a second goat; a second goat which develops from a reconstructed embryo or is descended from a goat which developed from a reconstructed embryo; or a second goat developed from a reconstructed embryo, or descended from a goat which developed from a reconstructed embryo, which was formed from genetic material from the same animal, an animal of the same genotype, or same cell line, which supplied the genetic material for the first goat. In a preferred embodiment, a first transgenic goat which develops from the reconstructed embryo can be mated with a second transgenic goat which developed from a reconstructed embryo and which contains a different transgene that the first transgenic goat.

In a preferred embodiment, the goat is a male goat. In other preferred embodiments, the goat is a female goat. A female goat can be induced to lactate and milk can be obtained from the goat.

In a preferred embodiment: a product, e.g., a protein, e.g., a recombinant protein, e.g., a human protein, is recovered from the goat; a product, e.g., a protein, e.g., a human protein, is recovered from the milk, urine, hair, blood, skin or meat of the goat.

In a preferred embodiment, the genome of the genetically engineered somatic cell includes a transgenic sequence. The transgenic sequence can be any of: a heterologous transgene, e.g., a human transgene; a knockout, knockin or other event which disrupts the expression of a mammalian gene; a sequence which encodes a protein, e.g., a human protein; a heterologous promoter; a heterologous sequence under the control of a promoter, e.g., a caprine or heterologous promoter. The transgenic sequence can encode any product of interest such as a protein, polypeptide or peptide.

In a preferred embodiment, the transgenic sequence encodes any of: a hormone, an immunoglobulin, a plasma protein, and an enzyme. The transgenic sequence can encode any protein whose expression in the transgenic mammal is desired, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin In, insulin, prolactin, and α1-antitrypsin.

In a preferred embodiment, the transgenic sequence encodes a human protein.

In a preferred embodiment, the transgenic sequence is under the control of a promoter, e.g., a caprine or heterologous promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be, e.g., any of: a casein promoter, a beta lactoglobulin promoter, a whey acid protein promoter and a lactalbumin promoter.

In a preferred embodiment, a nucleic acid has been introduced into the genome of the genetically engineered somatic cell. The nucleic acid sequence can be any of: a heterologous transgene, e.g., a human transgene; a knockout, knockin or other event which disrupts the expression of a mammalian gene; a sequence which encodes a protein, e.g., a human protein; a heterologous promoter; a heterologous sequence under the control of a promoter, e.g., a caprine or heterologous promoter. The transgenic sequence can encode any product of interest such as a protein, polypeptide or peptide.

In a preferred embodiment, the nucleic acid encodes any of: a hormone, an immunoglobulin, a plasma protein, and an enzyme. The nucleic acid sequence can encode any protein whose expression in the transgenic mammal is desired, e.g., any of: α-1 proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and α1-antitrypsin.

In a preferred embodiment, the nucleic acid sequence encodes a human protein.

In a preferred embodiment, the nucleic acid sequence is under the control of a promoter, e.g., a caprine or heterologous promoter. The promoter can be a tissue-specific promoter. The tissue specific promoter can be any of: milk-specific promoters; blood-specific promoters; muscle-specific promoters; neural-specific promoters; skin-specific promoters; hair-specific promoters; and urine-specific promoters. The milk-specific promoter can be, e.g., any of: a casein promoter, a beta lactoglobulin promoter, a whey acid protein promoter and a lactalbumin promoter.

In another aspect, the invention features a kit. The kit includes a reconstructed embryo which is in the 2 to 8 cell stage. In a preferred embodiment, the kit further includes instructions for producing a mammal, e.g., an embryonic, fetal or postnatal mammal.

In another aspect, the invention features a kit which includes a later stage embryo, e.g., an embryo after the 8 cell stage, or a fetus, obtained, e.g., by the methods described herein.

As used herein, the term "functional enucleation" refers to a process of rendering the endogenous genome of a cell, e.g., an oocyte, incapable of functioning, e.g., replicating and/or synthesizing DNA. Such an oocyte is referred to herein as a "functionally enucleated oocyte".

The terms protein, polypeptide and peptide are used interchangeably herein.

As used herein, the term "transgenic sequence" refers to a nucleic acid sequence (e.g., encoding one or more human proteins), which is inserted by artifice into a cell. The transgenic sequence, also referred to herein as a transgene, becomes part of the genome of an animal which develops in whole or in part from that cell. In embodiments of the invention, the transgenic sequence is integrated into the chromosomal genome. If the transgenic sequence is integrated into the genome it results, merely by virtue of its insertion, in a change in the nucleic acid sequence of the genome into which it is inserted. A transgenic sequence can be partly or entirely species-heterologous, i.e., the transgenic sequence, or a portion thereof, can be from a species which is different from the cell into which it is introduced. A transgenic sequence can be partly or entirely species-homologous, i.e., the transgenic sequence, or a portion thereof, can be from the same species as is the cell into which it is introduced. If a transgenic sequence is homologous (in the sequence sense or in the species-homologous sense) to an endogenous gene of the cell into which it is introduced, then the transgenic sequence, preferably, has one or more of the following characteristics: it is designed for insertion, or is inserted, into the cell's genome in such a way as to alter the sequence of the genome of the cell into which it is inserted (e.g., it is inserted at a location which differs from that of the endogenous gene or its insertion results in a change in the sequence of the endogenous endogenous gene); it includes a mutation, e.g., a mutation which results in misexpression of the transgenic sequence; by virtue of its insertion, it can result in misexpression of the gene into which it is inserted, e.g., the insertion can result in a knockout of the gene into which it is inserted. A transgenic sequence can include one or more transcriptional regulatory sequences and any other nucleic acid sequences, such as introns, that may be necessary for a desired level or pattern of expression of a selected nucleic acid, all operably linked to the selected nucleic acid. The transgenic sequence can include an enhancer sequence and or sequences which allow for secretion.

The terms "reconstructed embryo", "reconstituted embryo", "nuclear transfer unit" and "nuclear transfer embryo" are used interchangeably herein.

As used herein, the term "normal goat" refers to a goat which did not develop from a reconstructed embryo.

A "naturally derived oocyte" is one which is allowed to reach a selected stage, e.g., metaphase II or more preferably telophase, by culturing under natural conditions, e.g., in vivo. The term "natural conditions" means the absence of treatment of the oocyte with exogenously added chemicals, e.g., ethanol, to affect the stage of meiosis. In preferred embodiments, a naturally matured preparation can include metaphase II, telophase or both stages. The inventors have discovered that naturally matured oocytes are preferable to those which have been chemically induced.

Other features and advantages of the invention will be apparent from the following description and from the claims.

### Detailed Description of the Invention

### Sources of Somatic Genomes:

### Somatic Cells

Somatic cells can supply the genome for producing a reconstructed embryo in the methods described herein. The term "somatic cell", as used herein, refers to a differentiated cell. The cell can be a somatic cell or a cell that is committed to a somatic cell lineage. Alternatively, any of the methods and animals described herein can utilize a diploid stem cell that gives rise to a germ cell in order to supply the genome for producing a reconstructed embryo.

The somatic cell can be from an animal or from a cell culture. If taken from an animal, the animal can be at any stage of development, e.g., an embryo, a fetus or an adult. Embryonic cells are preferred. Embryonic cells can include embryonic stem cells as well as embryonic cells committed to a somatic cell lineage. Such cells can be obtained from the endoderm, mesoderm or ectoderm of the embryo. Preferably, the embryonic cells are committed to somatic cell lineage. Embryonic cells committed to a somatic cell lineage refer to cells isolated on or after day 10 of embryogenesis. However, cells can be obtained prior to day ten of embryogenesis. If a cell line is used as a source of a chromosomal genome, primary cells are preferred. The term "primary cell line" as used herein includes primary cell lines as well as primary-derived cell lines.

Suitable somatic cells include fibroblasts (e.g., primary fibroblasts, e.g., embryonic primary fibroblasts), muscle cells (e.g., myocytes), cumulus cells, neural cells, and mammary cells. Other suitable cells include hepatocytes and pancreatic islets. Preferably, the somatic cell is an embryonic somatic cell, e.g., a cell isolated on or after day 10 of embryogenesis. The genome of the somatic cells can be the naturally occurring genome, e.g., for the production of cloned mammals, or the genome can be genetically altered to comprise a transgenic sequence, e.g., for the production of transgenic cloned mammals.

Somatic cells can be obtained by, for example, dissociation of tissue, e.g., by mechanical (e.g., chopping, mincing) or enzymatic means (e.g., trypsinization) to obtain a cell suspension and then by culturing the cells until a confluent monolayer is obtained. The somatic cells can then be harvested and prepared for cryopreservation, or maintained as a stock culture. The isolation of caprine somatic cells, e.g., fibroblasts, is described herein.

The somatic cell can be a quiescent or non-quiescent somatic cell. "Non-quiescent", as used herein, refers to a cell in mitotic cell cycle. The mitotic cell cycle has four distinct phases, G₁, S, G₂ and M. The beginning event in the cell cycle, called START, takes place during the G₁ phase. "START" as used herein refers to early G₁ stage of the cell cycle prior to the commitment of a cell to proceeding through the cell cycle. For example, 1, 2, 3, 4, 5, 6, 7, 8, 9, 10 up to 11 hours after a cell enters the G₁ stage, the cell is considered prior to START. The decision as to whether the cell will undergo another cell cycle is made at START. Once the cell has passed through START, it passes through the remainder of the G₁ phase (i.e., the pre-DNA synthesis stage). The S phase is the DNA synthesis stage, which is followed by the G₂ phase, the stage between synthesis and mitosis. Mitosis takes place during the M phase. If at START, the cell does not undergo another cell cycle, the cell becomes quiescent. In addition, a cell can be induced to exit the cell cycle and become quiescent A "quiescent" cell, also referred to as a cell in G₀ phase, refers to a cell which is not in any of the four phases of the cell cycle. Preferably, the somatic cell is a cell in the G₀ phase or the G₁ phase of the mitotic cell cycle.

Using donor somatic cells at certain phases of the cell cycle, e.g., G₀ or G₁ phase, can allow for synchronization between the oocyte and the genome of the somatic cell. For example, reconstruction of an oocyte in metaphase II by introduction of a nucleus of a somatic cell in G₀ or G₁, e.g., by simultaneous activation and fusion, can mimic the events occurring during fertilization. By way of another example, an oocyte in telophase II fused, e.g., by simultaneous activation and fusion, with the genome of a somatic cell in G₁ prior to START, provides a synchronization of cell cycle between the oocyte and donor nuclei.

Methods of determining which phase of the cell cycle a cell is in are known. For example, as described below in the Examples, various markers are present at different stages of the cell cycle. Such markers can include cyclins D 1, 2, 3 and proliferating cell nuclear antigen (PCNA) for G₁, and BrDu to detect DNA synthetic activity. In addition, cells can be induced to enter the G₀ stage by culturing the cells on serum-deprived medium. Alternatively, cells in G₀ stage an be induced to enter the cell cycle, i.e., at G₁ stage, by serum activation.

The donor cells can be obtained from a mammal, e.g., an embryonic, fetal or adult mammal, or from a culture system, e.g., a synchronous culture system. For example, the donor cell can be selected from a culture system which contains at least a majority of donor cells (e.g., 50%, 55%, 60%, 65%, 70%, 75%, 80%, 85%, 90% or more) in a specific stage of the mitotic cell cycle.

### Sources of Genetically Engineered Somatic Cells:

### Transgenic Mammals

Methods for generating non-human transgenic mammals which can be used as a source of somatic cells in the invention are known in the art. Such methods can involve introducing DNA constructs into the germ line of a mammal to make a transgenic mammal. For example, one or several copies of the construct may be incorporated into the genome of a mammalian embryo by standard transgenic techniques.

Although goats are a preferred source of genetically engineered somatic cells, other non-human mammals can be used. Preferred non-human mammals are ruminants, e.g., cows, sheep, camels or goats. Goats of Swiss origin, e.g., the Alpine, Saanen and Toggenburg breed goats, are useful in the methods described herein. Additional examples of preferred non-human animals include oxen, horses, llamas, and pigs. The mammal used as the source of genetically engineered cells will depend on the transgenic mammal to be obtained by the methods of the invention as, by way of example, a goat genome should be introduced into a goat functionally enucleated oocyte.

Preferably, the somatic cells for use in the invention are obtained from a transgenic goat. Methods of producing transgenic goats are known in the art. For example, a transgene can be introduced into the germline of a goat by microinjection as described, for example, in Ebert et al. (1994) *Bio*/*Technology* 12:699, hereby incorporated by reference.

Other transgenic non-human animals to be used as a source of genetically engineered somatic cells can be produced by introducing a transgene into the germline of the non-human animal. Embryonal target cells at various developmental stages can be used to introduce transgenes. Different methods are used depending on the stage of development of the embryonal target cell. The specific line(s) of any animal used to practice this invention are selected for general good health, good embryo yields, good pronuclear visibility in the embryo, and good reproductive fitness. In addition, the haplotype is a significant factor.

### Transfected Cell Lines

Genetically engineered somatic cells for use in the invention can be obtained from a cell line into which a nucleic acid of interest, e.g., a nucleic acid which encodes a protein, has been introduced.

A construct can be introduced into a cell via conventional transformation or transfection techniques. As used herein, the terms "transfection" and "transformation" include a variety of techniques for introducing a transgenic sequence into a host cell, including calcium phosphate or calcium chloride coprecipitation, DEAE-dextrane-mediated transfection, lipofection, or electroporation. In addition, biological vectors, e.g., viral vectors can be used as described below. Suitable methods for transforming or transfecting host cells can be found in Sambrook et al., *Molecular Cloning: A Laboratory Manuel. 2*^{*nd*} *ed., Cold Spring Harbor Laboratory,* (Cold Spring Harbor Laboratory Press, Cold Spring Harbor, NY, 1989), and other suitable laboratory manuals.

Two useful approaches are electroporation and lipofection. Brief examples of each are described below.

The DNA construct can be stably introduced into a donor somatic cell line by electroporation using the following protocol: somatic cells, e.g., fibroblasts, e.g., embryonic fibroblasts, are resuspended in PBS at about 4 x 10⁶ cells/ml. Fifty micorgrams of linearized DNA is added to the 0.5 ml cell suspension, and the suspension is placed in a 0.4 cm electrode gap cuvette (Biorad). Electroporation is performed using a Biorad Gene Pulser electroporator with a 330 volt pulse at 25 mA, 1000 microFarad and infinite resistance. If the DNA construct contains a Neomyocin resistance gene for selection, neomyocin resistant clones are selected following incubation with 350 microgram/ml of G418 (GibcoBRL) for 15 days.

The DNA construct can be stably introduced into a donor somatic cell line by lipofection using a protocol such as the following: about 2 x 10⁵ cells are plated into a 3.5 cmiameter well and transfected with 2 micrograms of linearized DNA using LipfectAMINE™ (GibcoBRL). Forty-eight hours after transfection, the cells are split 1:1000 and 1:5000 and, if the DNA construct contains a neomyosin resistance gene for selection, G418 is added to a final concentration of 0.35 mg/ml. Neomyocin resistant clones are isolated and expanded for cyropreservation as well as nuclear transfer.

### Tissue-Specific Expression of Proteins

It is often desirable to express a protein, e.g., a heterologous protein, in a specific tissue or fluid, e.g., the milk, of a transgenic animal. The heterologous protein can be recovered from the tissue or fluid in which it is expressed. For example, it is often desirable to express the heterologous protein in milk. Methods for producing a heterologous protein under the control of a milk specific promoter are described below. In addition, other tissue-specific promoters, as well as, other regulatory elements, e.g., signal sequences and sequence which enhance secretion of non-secreted proteins, are described below.

### Milk Specific Promoters

Useful transcriptional promoters are those promoters that are preferentially activated in mammary epithelial cells, including promoters that control the genes encoding milk proteins such as caseins, beta lactoglobulin (Clark et al., (1989) Bio/Technology 7: 487-492), whey acid protein (Gordon et al. (1987) Bio/Technology 5:1183-1187), and lactalbumin (Soulier et al., (1992) FEBS Letts. 297:13). Casein promoters may be derived from the alpha, beta, gamma or kappa casein genes of any mammalian species; a preferred promoter is derived from the goat beta casein gene (DiTullio, (1992) Bio/Technology 10:74-77). Milk-specific protein promoter or the promoters that are specifically activated in mammary tissue can be derived from cDNA or genomic sequences. Preferably, they are genomic in origin.

DNA sequence information is available for the mammary gland specific genes listed above, in at least one, and often in several organisms. See, e.g., Richards et al., J. Biol. Chem. 256, 526-532 (1981) (α-lactalbumin rat); Campbell et al., Nucleic Acids Res. 12, 8685-8697 (1984) (rat WAP); Jones et al., J. Biol. Chem. 260, 7042-7050 (1985) (rat β-casein); Yu-Lee & Rosen, J. Biol. Chem. 258, 10794-10804 (1983) (rat γ-casein); Hall, Biochem. J. 242, 735-742 (1987) ( α-lactalbumin human); Stewart, Nucleic Acids Res. 12, 389 (1984) (bovine αs1 and κ casein cDNAs); Gorodetsky et al., Gene 66, 87-96 (1988) (bovine β casein); Alexander et al., Eur. J. Biochem. 178, 395401(1988) (bovine κ casein); Brignon et al., FEBS Lett. 188, 48-55 (1977) (bovine αS2 casein); Jamieson et al., Gene 61, 85-90 (1987), Ivanov et al., Biol. Chem. Hoppe-Seyler 369, 425-429 (1988), Alexander et al., Nucleic Acids Res. 17, 6739 (1989) (bovine β lactoglobulin); Vilotte et al., Biochimie 69, 609-620 (1987) (bovine α-lactalbumin). The structure and function of the various milk protein genes are reviewed by Mercier & Vilotte, J. Dairy Sci. 76, 3079-3098 (1993) (incorporated by reference in its entirety for all purposes). If additional flanking sequence are useful in optimizing expression of the heterologous protein, such sequences can be cloned using the existing sequences as probes. Mammary-gland specific regulatory sequences from different organisms can be obtained by screening libraries from such organisms using known cognate nucleotide sequences, or antibodies to cognate proteins as probes.

### Signal Sequences

Useful signal sequences are milk-specific signal sequences or other signal sequences which result in the secretion of eukaryotic or prokaryotic proteins. Preferably, the signal sequence is selected from milk-specific signal sequences, i.e., it is from a gene which encodes a product secreted into milk. Most preferably, the milk-specific signal sequence is related to the milk-specific promoter used in the construct, which are described below. The size of the signal sequence is not critical. All that is required is that the sequence be of a sufficient size to effect secretion of the desired recombinant protein, e.g., in the mammary tissue. For example, signal sequences from genes coding for caseins, e.g., alpha, beta, gamma or kappa caseins, beta lactoglobulin, whey acid protein, and lactalbumin can be used. A preferred signal sequence is the goat β-casein signal sequence.

Signal sequences from other secreted proteins, e.g., proteins secreted by kidney cells, pancreatic cells or liver cells, can also be used. Preferably, the signal sequence results in the secretion of proteins into, for example, urine or blood.

### Amino-Terminal Regions of Secreted Proteins

A non-secreted protein can also be modified in such a manner that it is secreted such as by inclusion in the protein to be secreted of all or part of the coding sequence of a protein which is normally secreted. Preferably the entire sequence of the protein which is normally secreted is not included in the sequence of the protein but rather only a sufficient portion of the amino terminal end of the protein which is normally secreted to result in secretion of the protein. For example, a protein which is not normally secreted is fused (usually at its amino terminal end) to an amino terminal portion of a protein which is normally secreted.

In one aspect, the protein which is normally secreted is a protein which is normally secreted in milk. Such proteins include proteins secreted by mammary epithelial cells, milk proteins such as caseins, beta lactoglobulin, whey acid protein , and lactalbumin. Casein proteins include alpha, beta, gamma or kappa casein genes of any mammalian species. A preferred protein is beta casein, e.g., goat beta casein. The sequences which encode the secreted protein can be derived from either cDNA or genomic sequences. Preferably, they are genomic 5 in origin, and include one or more introns.

### Other Tissue-Specific Promoters

Other tissue-specific promoters which provide expression in a particular tissue can be used. Tissue specific promoters are promoters which are expressed more strongly in a particular tissue than in others. Tissue specific promoters are often expressed essentially exclusively in the specific tissue.

Tissue-specific promoters which can be used include: a neural-specific promoter, e.g., nestin, Wnt-1, Pax-1, Engrailed-1, Engrailed-2, Sonic hedgehog; a liver-specific promoter, e.g., albumin, alpha-1 antirypsin; a muscle-specific promoter, e.g., myogenin, actin, MyoD, myosin; an oocyte specific promoter, e.g., ZP1, ZP2, ZP3; a testes-specific promoter, e.g., protamin, fertilin, synaptonemal complex protein-1; a blood-specific promoter, e.g., globulin, GATA-1, porphobilinogen deaminase; a lung-specific promoter, e.g., surfactant protein C; a skin- or wool-specific promoter, e.g., keratin, elastin; endothelium-specific promoters, e.g., Tie-1, Tie-2; and a bone-specific promoter, e.g., BMP.

In addition, general promoters can be used for expression in several tissues. Examples of general promoters include β-actin, ROSA-21, PGK, FOS, c-myc, Jun-A, and Jun-B.

### DNA Constructs

A cassette which encodes a heterologous protein can be assembled as a construct which includes a promoter for a specific tissue, e.g., for mammary epithelial cells, e.g., a casein promoter, e.g., a goat beta casein promoter, a milk-specific signal sequence, e.g., a casein signal sequence, e.g., a β-casein signal sequence, and a DNA encoding the heterologous protein.

The construct can also include a 3' untranslated region downstream of the DNA sequence coding for the non-secreted protein. Such regions can stabilize the RNA transcript of the expression system and thus increases the yield of desired protein from the expression system. Among the 3' untranslated regions useful in the constructs for use in the invention are sequences that provide a poly A signal. Such sequences may be derived, e.g., from the SV40 small t antigen, the casein 3' untranslated region or other 3' untranslated sequences well known in the art. In one aspect, the 3' untranslated region is derived from a milk specific protein. The length of the 3' untranslated region is not critical but the stabilizing effect of its poly A transcript appears important in stabilizing the RNA of the expression sequence.

Optionally, the construct can include a 5' untranslated region between the promoter and the DNA sequence encoding the signal sequence. Such untranslated regions can be from the same control region from which promoter is taken or can be from a different gene, e.g., they may be derived from other synthetic, semi-synthetic or natural sources. Again their specific length is not critical, however, they appear to be useful in improving the level of expression.

The construct can also include about 10%, 20%, 30%, or more of the N-terminal coding region of a gene preferentially expressed in mammary epithelial cells. For example, the N-terminal coding region can correspond to the promoter used, e.g., a goat β-casein N-terminal coding region.

The construct can be prepared using methods known in the art. The construct can be prepared as part of a larger plasmid. Such preparation allows the cloning and selection of the correct constructions in an efficient manner. The construct can be located between convenient restriction sites on the plasmid so that they can be easily isolated from the remaining plasmid sequences for incorporation into the desired mammal.

### Heterologous Proteins

Transgenic sequences encoding heterologous proteins can be introduced into the germline of a non-human mammal or can be transfected into a cell line to provide a source of genetically engineered somatic cells as described above.

The protein can be a complex or multimeric protein, e.g., a homo- or heteromultimer, e.g., proteins which naturally occur as homo- or heteromultimers, e.g., homo- or hetero- dimers, trimers or tetramers. The protein can be a protein which is processed by removal, e.g., cleavage, of N-terminus, C-terminus or internal fragments. Even complex proteins can be expressed in active form. Protein encoding sequences which can be introduced into the genome of mammal, e.g., goats, include glycoproteins, neuropeptides, immunoglobulins, enzymes, peptides and hormones. The protein may be a naturally occurring protein or a recombinant protein, e.g., a fragment, fusion protein, e.g., an immunoglogulin fusion protein, or mutien. It may be human or non-human in origin. The heterologous protein may be a potential therapeutic or pharmaceutical agent such as, but not limited to: alpha-1 proteinase inhibitor, alpha-1 antitrypsine, alkaline phosphatase, angiogenin, antithrombin III, any of the blood clotting factors including Factor VIII, Factor IX, and Factor X chitinase, erythropoietin, extracellular superoxide dismutase, fibrinogen, glucocerebrosidase, glutamate decarboxylase, human growth factor, human serum albumin, immunoglobulin, insulin, myelin basic protein, proinsulin, prolactin, soluble CD4 or a component or complex thereof, lactoferrin, lactoglobulin, lysozyme, lactalbumin, tissue plasminogen activator or a variant thereof.

Immunoglobulins are particularly preferred heterologous protiens. Examples of immunoglobulins include IgA, IgG, IgE, IgM, chimeric antibodies, humanized antibodies, recombinant antibodies, single chain antibodies and antibody-protein fusions.

Nucleotide sequence information is available for several of the genes encoding the heterologous proteins listed above, in at least one, and often in several organisms. *See e.g.,* Long et al. (1984) *Biochem.* 23(21):4828-4837 (aplha-1 antitrypsin); Mitchell et al. (1986) *Prot. Natl. Acad. Sci USA* 83:7182-7186 (alkaline phosphatase); Schneider et al. (1988) *EMBO J.* 7(13):4151-4156 (angiogenin); Bock et al. (1988) *Biochem.* 27(16):6171-6178 (antithrombin III); Olds et al. (1991) *Br. J. Haematol.* 78(3):408-413 (antithrombin III); Lin et al. (1985) *Proc. Natl. Acad. Sci. USA* 82(22):7580-7584 (erythropoeitin); U.S. Patent No. 5,614,184 (erythropoietin); Horowitz et al. (1989) *Genomics* 4(1):87-96 (glucocerebrosidase); Kelly et al. (1992) *Ann. Hum. Genet.* 56(3):255-265 (glutamte decarboxylase); U.S. Patent No. 5,707,828 (human serum albumin); U.S. Patent No. 5,652,352 (human serum albumin); Lawn et al. (1981) *Nucleic Acid Res.* 9(22):6103-6114 (human serum albumin); Kamholz et al. (1986) *Prot. Natl. Acad. Sci. USA* 83(13):4962-4966 (myelin basic protein); Hiraoka et al. (1991) *Mol. Cell Endocrinol.* 75(1):71-80 (prolactin); U.S. Patent No. 5,571,896 (lactoferrin); Pennica et al. (1983) *Nature* 301(5897):214-221 (tissue IIIIplasminogen activator); Sarafanov et al. (1995) *Mol. Biol.* 29:161-165, the contents of which are incorporated herein by reference.

### Oocytes

Oocytes for use in the invention include oocytes in metaphase II stage of meiotic cell division, e.g., oocytes arrested in metaphase II, and telophase stage of meiotic division, e.g., telophase I or telophase II. Oocytes in metaphase II contain one polar body, whereas oocytes in telophase can be identified based on the presence of a protrusion of the plasma membrane from the second polar body up to the formation of a second polar body. In addition, oocytes in metaphase II can be distinguished from oocytes in telophase II based on biochemical and/or developmental distinctions. For example, oocytes in metaphase II can be in an arrested state, whereas oocytes in telophase are in an activated state. Preferably, the oocyte is a caprine oocyte.

Occytes can be obtained at various times during a goat's reproductive cycle. For example, at given times during the reproductive cycle, a significant percentage of the oocytes, e.g., about 55%, 60%, 65%, 70%, 75%, 80% or more, are oocytes in telophase. These oocytes are naturally matured oocytes. In addition, oocytes at various stages of the cell cycle can be obtained and then induced *in vitro* to enter a particular stage of meiosis. For example, oocytes cultured on serum-starved medium become arrested in metaphase. In addition, arrested oocytes can be induced to enter telophase by serum activation. Thus, oocytes in telophase can be easily obtained for use in the invention.

Oocytes can be matured *in vitro* before they are used to form a reconstructed embryo. This process usually requires collecting immature oocytes from mammalian ovaries, e.g., a caprine ovary, and maturing the oocyte in a medium prior to enucleation until the oocyte reaches the desired meiotic stage, e.g., metaphase or telophase. In addition, oocytes that have been matured *in vivo* can be used to form a reconstructed embryo.

Oocytes can be collected from a female mammal during superovulation. Briefly, oocytes, e.g., caprine oocytes, can be recovered surgically by flushing the oocytes from the oviduct of the female donor. Methods of inducing superovulation in goats and the collection of caprine oocytes is described herein.

Preferably, the meiotic stage of the oocyte, e.g., metaphase II or telophase II, correlates to the stage of the cell cycle of the donor somatic cell. The correlation between the meiotic stage of the oocyte and the mitotic stage of the cell cycle of the donor somatic cell is referred to herein as "synchronization". For example, reconstruction of an oocyte in metaphase II by introduction of a nucleus of a somatic cell in G₀ or G₁ e.g., by simultaneous activation and fusion, can mimic the events occurring during fertilization. By way of another example, an oocyte in telophase fused, e.g., by simultaneous activation and fusion, with the genome of a somatic cell in G₁ prior to START, provides a synchronization between the oocyte and the donor nuclei.

### Functional Enucleation

The donor oocyte, e.g., caprine oocyte, should be functionally enucleated such that the endogenous genome of the oocyte is incapable of functioning, e.g., replicating or synthesizing DNA. Methods of functionally enucleating an oocyte include: removing the genome from the oocyte (i.e., enucleation), e.g., such that the oocyte is devoid of nuclear genome; inactivating DNA within the oocyte, e.g., by irradiation (e.g., by X-ray irradiation, or laser irradiation); chemical inactivation, or the like.

### Enucleation

One method of rendering the genome of an oocyte incapable of functioning is to remove the genome from the oocyte (i.e., enucleation). A micropipette or needle can be inserted into the zona pellicuda in order to remove nuclear material from an oocyte. For example, metaphase II stage oocytes which have one polar body can be enucleated with a micropipette by aspirating the first polar body and adjacent cytoplasm surrounding the polar body, e.g., approximately 20%, 30%, 40%, 50%, 60% of the cytoplasm, which presumably contains the metaphase plate. Telphase stage oocytes which have two polar bodies can be enucleated with a micropipette or needle by removing the second polar body and surrounding cytoplasm, e.g., approximately 5%, 10%, 20%, 30%, 40%, 50%, 60% of cytoplasm. Specifically, oocytes in telophase stage can be enucleated at any point from the presence of a protrusion in the plasma membrane from the second polar body up to the formation of the second polar body. Thus, as used herein, oocytes which demonstrate a protrusion in the plasma membrane, usually with a spindle abutted to it, up to extrusion of the second polar body are considered to be oocytes in telophase. Alternatively, oocytes which have one clear and distinct polar body with no evidence of protrusion are considered to be oocytes in metaphase. Methods of enucleating an oocyte, e.g., a caprine oocyte, are described in further detail in the Examples.

### Irradiation

The oocyte can be functionally enucleated by inactivating the endogenous DNA of the oocyte using irradiation. Methods of using irradiation are known in the art and described, for example, in Bradshaw et al. (1995) *Molecul. Reprod. Dev.* 41:503-512, the contents of which is incorporated herein by reference.

### Chemical Inactivation

The oocyte can be functionally enucleated by chemically inactivating the endogenous DNA of the oocyte. Methods of chemically inactivating the DNA are known in the art. For example, chemical inactivation can be performed using the etopsoide-cycloheximide method as described in Fulkaj and Moore (1993) *Molecul. Reprod. Dev.* 34:427-430, the content of which are incorporated herein by reference.

### Introduction of a Functional Chromosomal Genome into an Oocyte

Methods described herein can include the introduction of a functional chromosomal genome into an oocyte, e.g., a functionally enucleated oocyte, e.g., an enucleated oocyte, to form a reconstructed embryo. The functional chromosomal genome directs the development of a cloned or transgenic animal which arises from the reconstructed embryo. Methods which result in the transfer of an essentially intact chromosomal genome to the oocyte can be used. Examples include fusion of a cell which contains the functional chromosomal genome with the oocyte and nuclear injection, i.e., direct transfer of the nucleus into the oocyte.

### Fusion

Fusion of the somatic cell with an oocyte can be performed by, for example, electrofusion, viral fusion, biochemical reagent fusion (e.g., HA protein), or chemical fusion (e.g., with polyethylene glycol (PEG) or ethanol).

Fusion of the somatic cell with the oocyte and activation can be performed simultaneously. For example, the nucleus of the somatic cell can be deposited within the zona pelliduca which contains the oocyte. The steps of fusing the nucleus with the oocyte and activation can then be performed simultaneously by, for example, applying an electric field. Methods of simultaneous fusion and activation of a somatic cell and an oocyte are described herein.

### Activation of a Recombinant Embryo

Activation refers to the beginning of embryonic development, e.g., replication and DNA synthesis. Activation can be induced by, for example, electric shock (e.g., in electrofusion), the use of ionophores, ethanol activation, or the oocyte can be obtained during a stage in which it is naturally activated, e.g., an oocyte in telophase.

### Electrofusion

A reconstructed embryo can be activated using electric shock, i.e., electrofusion. The use of electrofusion allows for the fusion of the somatic cell with the oocyte and activation to be performed simultaneously.

Chambers, such as the BTX 200 Embryomanipulation System, for carrying out electrofusion are commercially available from, for example, BTX, San Diego. Methods for performing electrofusion to fuse a somatic cell, e.g., a caprine somatic cell, and an oocyte, e.g., an enucleated oocyte, e.g., an enucleated caprine oocyte, are described herein.

### Ionophores

In addition, the reconstructed embryo can be activated by ionophore activation. Using an ionophore, e.g., a calcium ionophore, the calcium concentration across the membrane of the reconstructed embryo is changed. As the free calcium concentration in the cell increases, there is a decrease in phosphorylation of intracellular proteins and the oocyte is activated. Such methods of activation are described, for example, in U.S. Patent Number 5,496,720, the contents of which are incorporated by reference.

### Ethanol Activation

Prior to enucleation, an oocyte, e.g., an oocyte in metaphase II, can be activated with ethanol according to the ethanol activation treatment as described Bin Presicce and Yang *(1994) Mol. Reprod. Dev.* 37:61-68, and Bordignon and Smith (1998) *Mol. Reprod. Dev,* 49:29-36, the contents of which are incorporated herein by reference.

### Ooctyes in Telophase

Oocytes in telophase are generally already activated. Thus, these cells often naturally exhibit a decrease in calcium concentration which prevents fertilization and allows the embryo to develop.

### Transfer of Reconstructed Embryos

A reconstructed embryo of the invention can be transferred, e.g., implanted, to a recipient doe and allowed to develop into a cloned or transgenic mammal, e.g., a cloned or transgenic goat. For example, the reconstructed embryo can be transferred via the fimbria into the oviductal lumen of each recipient doe as described below in the Examples. In addition, methods of transferring an embryo to a recipient mammal are known in the art and described, for example, in Ebert et al. (1994) *Bio*/*Technology* 12:699.

The reconstructed embryo can be maintained in a culture until at least first cleavage (2-cell stage) up to blastocyst stage, preferably the embryos are transferred at 2-cell or 4 cell-stage. Various culture media for embryo development are known in the art. For example, the reconstructed embryo can be co-cultured with oviductal epithelial cell monolayer derived from the type of mammal to be provided by the invention. Methods of obtaining goat oviductal epithelial cells (GOEC), maintaining the cells in a co-culture are described in the Examples below.

### Purification of Proteins from Milk

The transgenic protein can be produced in milk at relatively high concentrations and in large volumes, providing continuous high level output of normally processed peptide that is easily harvested from a renewable resource. There are several different methods known in the art for isolation of proteins form milk.

Milk proteins usually are isolated by a combination of processes. Raw milk first is fractionated to remove fats, for example, by skimming, centrifugation, sedimentation (H.E. Swaisgood, Developments in Dairy Chemistry, I: Chemistry of Milk Protein, Applied Science Publishers, NY, 1982), acid precipitation (U.S. Patent No. 4,644,056) or enzymatic coagulation with rennin or chymotrypsin (Swaisgood, ibid.). Next, the major milk proteins may be fractionated into either a clear solution or a bulk precipitate from which the specific protein of interest may be readily purified.

French Patent No. 2487642 describes the isolation of milk proteins from skim milk or whey by membrane ultrafiltration in combination with exclusion chromatography or ion exchange chromatography. Whey is first produced by removing the casein by coagulation with rennet or lactic acid. U.S. Patent No. 4,485,040 describes the isolation of an alpha-lactoglobulin-enriched product in the retentate from whey by two sequential ultrafiltration steps. U.S. Patent No. 4,644,056 provides a method for purifying immunoglobulin from milk or colostrum by acid precipitation at pH 4.0-5.5, and sequential cross-flow filtration first on a membrane with 0.1 -.1.2 micrometer pore size to clarify the product pool and then on a membrane with a separation limit of 5 - 80 kd to concentrate it

Similarly, U.S. Patent No. 4,897,465 teaches the concentration and enrichment of a protein such as immunoglobulin from blood serum, egg yolks or whey by sequential ultrafiltration on metallic oxide membranes with a pH shift. Filtration is carried out first at a pH below the isoelectric point (pI) of the selected protein to remove bulk contaminants from the protein retentate, and next at a pH above the pI of the selected protein to retain impurities and pass the selected protein to the permeate. A different filtration concentration method is taught by European Patent No. EP 467 482 B 1 in which defatted skim milk is reduced to pH 3-4, below the pI of the milk proteins, to solubilize both casein and whey proteins. Three successive rounds of ultrafiltration or diafiltration then concentrate the proteins to form a retentate containing 15-20% solids of which 90% is protein. Alternatively, British Patent Application No. 2179947 discloses the isolation of lactoferrin from whey by ultrafiltration to concentrate the sample, followed by weak cation exchange chromatography at approximately a neutral pH. No measure of purity is reported. In PCT Publication No. WO 95/22258, a protein such as -lactoferrin is recovered from milk that has been adjusted to high ionic strength by the addition of concentrated salt, followed by cation exchange chromatography.

In all of these methods, milk or a fraction thereof is first treated to remove fats, lipids, and other particulate matter that would foul filtration membranes or chromatography media. The initial fractions thus produced may consist of casein, whey, or total milk protein, from which the protein of interest is then isolated.

PCT Patent Publication No. WO 94/19935 discloses a method of isolating a biologically active protein from whole milk by stabilizing the solubility of total milk proteins with a positively charged agent such as arginine, imidazole or Bis-Tris. This treatment forms a clarified solution from which the protein may be isolated, e.g., by filtration through membranes that otherwise would become clogged by precipitated proteins.

USSN 08/648,235 discloses a method for isolating a soluble milk component, such as a peptide, in its biologically active form from whole milk or a milk fraction by tangential flow filtration. Unlike previous isolation methods, this eliminates the need for a first fractionation of whole milk to remove fat and casein micelles, thereby simplifying the process and avoiding losses of recovery and bioactivity. This method may be used in combination with additional purification steps to further remove contaminants and purify the product, e.g., protein, of interest.

This invention is further illustrated by the following examples which in no way should be construed as being further limiting. The contents of all cited references (including literature references, issued patents, published patent applications, and co-pending patent applications) cited throughout this application are incorporated by reference.

### Examples

Donors and recipients used in the following examples were dairy goats of the following breeds (mixed or not): Alpine, Saanen, and Toggenburg. All goats were maintained at the Genzyme Transgenics farm in Charlton, Massachusetts. Collections and transfers were completed during the spring and early summer (off-season).

### Isolation of Caprine Somatic Cells

Caprine fetal fibroblast cell lines used as karyoplast donors were derived from six day 35-40 fetuses produced by artificially inseminating non-transgenic does with fresh collected semen from a transgenic antithrombin III (ATIII) founder buck. An ATIII cell line was chosen since it provides a well characterized genetic marker to the somatic cell lines, and it targets high level expression of a complex glycosylated protein (ATIII) in the milk of lactating does. Three fetuses which were derived from the semen of the transgenic ATIII buck were surgically removed at day 40 post coitus and placed in equilibrated Ca⁺⁺/Mg⁺⁺-free phosphate buffered saline (PBS). Cell suspensions were prepared by mincing and digesting fetal tissue in 0.025% trypsin/0.5 mM EDTA at 37°C for ten minutes. Cells were washed with equilbrated Medium 99™ (M199)(Gibco) + 10% Fetal Bovine Serum (FBS) supplemented with nucleosides, 0.1 mM 2-mercaptoethanol, 2 mM L-glutamine, 1% penicillin/streptomycin (10,000 I.U. each/ml) (fetal cell medium), and cultured in 25 cm² flasks. The cultures were re-fed 24 hours later with equilibrated fetal cell medium. A confluent monolayer of primary fetal cells was harvested by trypsinization on day four by washing the monolayer twice with Ca⁺⁺/Mg⁺⁺-free PBS, followed by incubation with 0.025% trypsin/0.5 mM EDTA at 38°C for 7 minutes.

Cells potentially expressing ATIII were then prepared for cryopreservation, or maintained as stock cultures.

### Sexing and Genotyping of Donor Cell Lines

Genomic DNA was isolated from fetal head tissue for ATIII donor karyoplasts by digestion with proteinase K followed by precipitation with isopropanol as described in Laird et al. (1991) *Nucleic Acid Res.* 19:4293, and analyzed by polymerase chain reaction (PCR) for the presence of human Antithrombin III (ATIII) sequences as well as for sexing. The ATIII sequence is part of the BC6 construct (Goat Beta-Casein - human ATIII cDNA) used to generate the ATIII transgenic line as described in Edmunds et al. (1998) *Blood* 91:4561-4571. The human ATIII sequencewas detected by amplification of a 367 bp sequence with oligonucleotides GTC11 and GTC12 (see below). For sexing, the zfX/zfY primer pair was used (see below) giving rise to a 445 bp (zfX)/447 bp (zfy) doublet. Upon digestion with the restriction enzyme SacI (New England Biolabs), the zfX band was cut into two small fragments (272 and 173 bp). Males were identified by the presence of the uncut 447 bp zfY band.

For the PCR reactions, approximately 250 ng of genomic DNA was diluted in 50 ml of PCR buffer (20 mM Tris pH 8.3, 50 mM KCl and 1.5 mM MgCl₂, 0.25 mM deoxynucleotide triphosphates, and each primer at a concentration of 600 mM) with 2.5 units of Taq polymerase and processed using the following temperature program:

| | | | |
|---|---|---|---|
| 1 cycle at | 94°C | 60 seconds | |
| 5 cycles at | 94°C | 30 seconds | |
| | | 58°C | 45 seconds |
| | | 74°C | 45 seconds |
| 30 cycles at | 94°C | 30 seconds | |
| | | 55°C | 30 seconds |
| | | 74°C | 30 seconds |

The following primer set was used to detect the human ATIII sequence:

GTC 11: CTCCATCAGTTGCTGGAGGGTGTCATTA (SEQ ID NO:1)

GTC 12: GAAGGTTTATCTTTTGTCCTTGCTGCTCA (SEQ ID NO:2)

The following primer set was used for sexing:

zfX: ATAATCACATGGAGAGCCACAAGC (SEQ ID N

zfY: GCACTTCTTTGGTATCTGAGAAAG (SEQ ID NO:4)

Two of the fetuses were identified to be male and were both negative for the ATIII sequence. Another fetus was identified as female and confirmed positive for the presence of the ATIII sequence.

### Preparation of Aim-Expressing Donor Cells for Embryo Reconstitution

A transgenic female line (CFF155-92-6) originating from a day 40 fetus was identified by PCR analyses, as described above, and used for all nuclear transfer manipulations. Transgenic fetal fibroblast cells were maintained in 25 cm² flasks with fetal cell medium, re-fed on day four following each passage, and harvested by trypsinization on day seven. From each passage, a new 25 cm² flasks was seeded to maintain the stock culture. Briefly, fetal cells were seeded in 4-well plates with fetal cell medium and maintained in culture (5% CO₂ at 39°C). Forty-eight hours later, the medium was replaced with fresh fetal cell medium containing 0.5% FBS. The culture was re-fed every 48-72 hours over the next seven days with fresh fetal cell medium containing 0.5% FBS. On the seventh day following first addition of fetal cell medium (0.5% FBS), somatic cells used as karyoplast donors were harvested by trypsinization as previously described. The cells were resuspended in equilibrated M199+10% FBS supplemented with 2mM L-glutamine, 1% penicillin/streptomycin (10,000 I.U. each/ml) one to three hours prior to fusion to the enucleated oocytes.

### Karyotyping of Cell Lines

The clonal lines were further evaluated by karyotyping to determine gross chromosomal abnormalities in the cell lines. Cells were induced to arrest at metaphase by incubation with 0.02 µg/ml of Demecolcine (Sigma) for 12 hours. After trypsinization, the resulting pellet was suspended in a hypotonic solution of 75 mM KCl in water and incubated at 37°C for 20 minutes. Cells were fixed for 5 minutes each time in 3 changes of ice-cold acetic acid-methanol (1:3) solution before drops of the cell suspension were placed in pre-washed microscopic slides. Following air-drying, chromosome preparations were stained with 3% Giemsa stain (Sigma) in PBS for 10 minutes. The chromosome spreads were counted for each cell line at 1000x magnification under oil immersion.

### Immunohistochemical Analysis

Antibodies specific for vimentin (Sigma) and pan-cytokeratin (Sigma) were used to characterize and confirm the morphology of the cell lines. Cells were plated in sterile gelatin coated cover slips to 75% confluency and fixed in 2% paraformaldehyde with 0.05% saponin for 1 hour. Cells were incubated in 0.5% PVP in PBS (PBS/PVP) with primary antibodies for 2 hours at 37 °C, rinsed with 3 changes of PBS/PVP at 10 minute intervals, and incubated for 1 hour in secondary antibodies conjugated with Cy3 and FITC respectively. Alkaline phosphatase (Sigma) activity of the cells was also performed to determine the presence or absence of undifferentiated cells, The cover slips were rinsed and subsequently mounted on glass slides with 50% glycerol inPBS/PVP with 10 µg/ml bisbenzimide (H-33342, Sigma) and observed under fluorescent microscopy.

Epithelial and fibroblast lines positive for vimentin and pan-cytokeratin, respectively, and negative for alkaline phosphatase activity were generated from the ATIII primary cultures. In the cell cultures, two morphologically distinct cell types were observed. Larger "fibroblast-like" cells stained positive for vimentin and smaller "epithelial-like" cells stained positive for pan-cytokeratin which coexisted in the primary cell cultures. The isolated fibroblast lines from ATIII showed a tendency to differentiate into epithelial-like cells when cultured for 3 days after reaching confluency. Subsequent passages induced selection against fibroblast cells giving rise to pure epithelial cells as confirmed by the lack of positive staining for vimentin. Senesces or possible cell cycle arrest was first observed at passage 28. These cells appear bigger in size (>30 µm) compared to the normally growing cells (15-25 µm) and can be maintained in culture in the absence of apparent mitotic activity for several months without loss of viability. Embryo reconstruction using nuclei from the arrested cells produced morula stage embyos suggesting reacquistion of mitotic activity.

### Donor Karyoplast Cell Cycle Synchronization and Characterization

Selected diploid transgenic female cell lines were propagated, passaged sequencially and cyrobanked as future karyoplast stock. Donor karyoplasts for nuclear transfer were seeded in 4 well plates and cultured for up to 48 hours in DMEM + 10% FBS or when cells reached 70-80% confluency. Subsequently, the cells were induced to exit growth phase and enter the quiescent stage (G₀) by serum deprivation for seven days using DMEM supplemented with 0.5% FBS to synchronize the cells. Following synchronization at G₀, a group of cells were induced to re-enter the cell cycle by resuspending the cells in M199 + 10% FBS up to three hours prior to karyoplast-cytoplast fusion to synchronize the cells at the early G₁ phase prior to START. A second group of cells were also released from the quiescent state and cultured in M199 + 10% FBS for 12 or 36 hours to synchronize cells at the S-phase. Cells were harvested by standard trypsinisation and resuspended in M199 + 10% FBS and electofased as karyoplasts donors within 1 hour.

The metaphase spreads from the transgenic cell lines carrying the ATIII construct at passage 5 was 81 % diploid and this did not alter significantly at passage 15 where 78% of the spreads were diploid.

Cell cycle synchrony was determined by immunohistochemical analysis using antibodies against cyclin D1, 2, 3 and PCNA (Oncogene Research Products) for the absence of the protein complex to indicate quiescence (G₀) or presence of the complex to indicate G₁ entry. Cells in the presumed S-phase of the cell cycle were identified by the presence of DNA synthetic activity using the thymidine analog 5-bromo 2'-deoxyuridine-5'triphospate (BrDu, Sigma) and streptavidin-Biotin BrDu staining kit (Oncogene Research Products).

Immunofluorescence analysis of cells subjected to the synchronization regimen demonstarted that following seven days of serum deprivation, 90% of the cells were negative for G₁ stage cyclins D 1, 2,,3 and PNCA, and were therefore in G₀ arrest. Restoration of the serum content to 10% for this line induced reentry into the cell cycle with approximately 74% of the cells reaching early G₁ within 3 hours following serum addition based on positive staining for cyclins D 1, 2, 3 and PCNA. Serum restoration for 12 to 36 hours showed that 89% of the cells were positive for BrDu indicating DNA synthetic activity. In this study, clonal lines generally responded differently to the serum synchronization regimen. An indirect relationship was observed where the rate of cell synchronization decreases with the increase in passage numbers. Further, as passage number increased the population doubling times decreased, each clonal cell line revealed a decreased sensitivity to serum synchronization of the cell cycle.

### Superovulation of Donor Goats and Oocyte Collection

Estrus was synchronized on day 0 by a 6 mg subcutaneous Norgestomet ear implant (Synchro-mate B). A single injection of prostaglandin (PGF2a)(Upjohn US) was administered on day 7. Starting on day 12, FSH (Folltropin-V, Vetrepharm, St Laurent, Quebec, Canada) was administered twice daily over four consecutive days. The ear implant was removed on day 14. Twenty-four hours following implant removal, the donor animals were mated several times to vasectomized males over a 48 hour interval. A single injection of GnRH (Rhone-Merieux US) was administered intramuscularly following the last FSH injection. Oocytes were recovered surgically from donor animals by mid-ventral laparotomy approximately 18 to 24 hours following the last mating, by flushing the oviduct with Ca⁺⁺/Mg⁺⁺ -free PBS prewarmed at 37°C. Oocytes were then recovered and cultured in equilibrated M199+10%FBS supplemented with 2mM L-glutamine, 1% penicillin/streptomycin (10,000 I.U. each/ml).

### Oocyte Enucleation

*In vivo* matured oocytes were collected from donor goats. Oocytes with attached cumulus cells or devoid of polar bodies were discarded. Cumulus-free oocytes were divided into two groups: oocytes with only one polar body evident (metaphase II stage) and the activated telophase II protocol (oocytes with one polar body and evidence of an extruding second polar body). Oocytes in telophase II were cultured in M199 + .10% FBS for 2 to 4 hours. Oocytes that had activated during this period, as evidenced by a first polar body and a partially extruded second polar body, were grouped as culture induced, calcium activated telophase II oocytes (Telophase II-Ca²⁺) and enucleated. Oocytes that had not activated were incubated for 5 minutes in PBS containing 7% ethanol prior to enucleation. Metaphase II stage oocytes (one polar body) were enucleated with a 25-30 micron glass pipette by aspirating the first polar body and adjacent cytoplasm surrounding the polar body (approximately 30% of the cytoplasm) presumably containing metaphase plate.

As discussed above, telophase stage oocytes were prepared by two procedures. Oocytes were intially incubated in phosphate buffered saline (PBS, Ca²⁺/Mg²⁺ free) supplemented with 5% FBS for 15 minutes and cultured in M199 + 10% FBS at 38°C for approximately three hours until the telophase spindle configuration or the extrusion of the second polar body was reached. All the oocytes that responded to the sequential culture under differential extracellular calcium concentration treatment were seperated and grouped as Telophase II-Ca²⁺. The other oocytes that did not respond were further incubated in 7% ethanol in M199 + 10% FBS for 5-7 minutes (Telophase II-ETOH) and cultured in M199 + 10% FBS at 38°C for another 3 hours until the telophase II spindle configuration was reached. Thereafter, the oocytes were incubated in 30-50 µl drops of M199 + 10% FBS conatining 5 µg/ml of cytochalasin-B for 10-15 minutes at 38°C. Oocytes were enucleated with a 30 micron (OD) glass pipette by aspirating the first polar body and approximately 30% of the adjacent cytoplasm containg the metaphase II or about 10% of the cytoplasm containing the telophase II spindle. After enucleation the oocytes were immediately reconstructed.

### Embryo Reconstruction

CFF155-92-6 somatic cells used as karyoplast donors were harvested on day 7 by trypsinizing (0.025% trypsin/0.5 mM EDTA)(Sigma) for 7 minutes. Single cells were resuspended in equilibrated M199+10% FBS supplemented with 2mM L-glutamine, penicillin/streptomycin. The donor cell injection was carried out in the same medium as for enucleation. Donor cells were graded into small, medium and large before selection for injection to enucleated cytoplasts. Small single cells (10-15 micron) were selected with a 20-30 micron diameter glass pipette. The pipette was introduced through the same slit of the zona made during enucleation and donor cells were injected between the zona pellucida and the ooplasmic membrane. The reconstructed embryos were incubated in M199 30-60 minutes before fusion and activation.

### Fusion and Activation

All reconstructed embryos (ethanol pretreatment or not) were washed in fusion buffer (0.3 M mannitol, 0.05 mM CaCl₂, 0.1 mM MgSO₄, 1 mM K₂HPO₄, 0.1 mM glutathione, 0.1 mg/ml BSA in distilled water) for 2 minutes before electrofusion. Fusion and activation were carried out at room temperature, in a chamber with two stainless steel electrodes 200 microns apart (BTX 200 Embryomanipulation System, BTX-Genetronics, San Diego, CA) filled with fusion buffer. Reconstructed embryos were placed with a pipette in groups of 3-4 and manually aligned so the cytoplasmic membrane of the recipient oocytes and donor CFF155-92-6 cells were parallel to the electrodes. Cell fusion and activation were simultaneously induced 32-42 hours post GnRH injection with an initial alignment/holding pulse of 5-10 V AC for 7 seconds, followed by a fusion pulse of 1.4 to 1.8 KV/cm DC for 70 microseconds using an Electrocell Manipulator and Enhancer 400 (BTX-Genetronics). Embryos were washed in fusion medium for 3 minutes, then they were transferred to M199 containing 5 µg/ml cytochalasin-B (Sigma) and 10% FBS and incubated for 1 hour. Embryos were removed from M199/cytochalasin-B medium and cocultured in 50 microliter drops of M199 plus 10% FBS with goat oviductal epithelial cells overlaid with paraffin oil. Embryo cultures were maintained in a humidified 39°C incubator with 5% CO₂ for 48 hours before transfer of the embryos to recipient does.

Reconstructed embryos at 1 hour following simultaneous activation and fusion with G₀,G₁ and S-phase karyoplasts all showed nuclear envelope breakdown (NEBD) and premature chromosome condensation (PCC) when the cytoplasts were at the arrested metaphase II stage. Subsequent nuclear envelope formation was observed to be at about 35% at 4 hour post activation. Oocytes reconstructed at telophase II stage showed that an average of 22% of oocytes observed at 1 hour post fusion of G₀ ,G₁ and S-phase karyoplast underwent NEBD and PCC, whereas the remaining oocytes have intact nuclear lamina surrounding the decondensing nucleus. No consistent nuclear morphology other than lack of, or the occurrence of NEBD and PCC was observed between the metaphase and two telophase reconstruction protocols employed. Differences became evident when cloned embryos were observed to have a higher incidence of advanced cleavage stages (8 to 32 blastomeres) when embryos were reconstructed with S-phase donor nuclei compared to when G₀ or G₁ stage karyoplasts were used (2 to 8 blastomeres) following culture *in vitro* for 36 to 48 hours. Fluorescent microscopy analysis showed that the nuclei of some of the rapidly dividing embryos were fragmented. Other embryos developed to the 32 to 64 cell stage within 3 days of culture before cleavage development was blocked. Analysis of blastomere and nuclei numbers of these embryos showed the failure of synchronous occurrence of cytokines and karyokinesis wherein blastomeres were either devoid or their corresponding nuclei or contained multiple nuclei. In contrast, morphologically normal looking embryos showed synchronous cytokinesis and karyokinesis.

### Goat Oviductal Epithelial Cells (GOEC)/Reconstructed Embryo Coculture

GOEC were derived from oviductal tissue collected during surgical oviductal flushing performed on synchronized and superovulated does. Oviductal tissue from a single doe was transferred to a sterile 15 ml polypropylene culture tube containing 5 ml of equilibrated M199, 10% FBS, 2 mM L-glutamine, penicillin/strepomycin. A single cell suspension was prepared by vortexing for 1 minute, followed by culture in a humidified 5% CO2 incubator at 38°C for up to one hour. The tube was vortexed a second time for one minute, then cultured an additional five minutes to allow debris to settle. The top four millimeters containing presumed single cells was transferred to a new 15 ml culture tube and centrifuged at 600x g for 7 minutes, at room temperature. The supernatant was removed, and the cell pellet resuspended in 8 ml of equilibrated GOEC medium. The GOEC were cultured in a 25 cm² flask, re-fed on day 3, and harvested by trypsinization on day six, as previously described. Monolayers were prepared weekly, from primary GOEC cultures, for each experiment. Cells were resuspended in GOEC medium at 5x10⁵/ml, and 50 microliter/well was seeded in 4-well plates (15mm). The medium was overlaid with 0.5 ml light paraffin oil, and the plates were cultured in a humidified 5% CO² incubator at 38°C. The cultures were re-fed on day two with 80% fresh equilibrated culture medium. All reconstructed embryos were cocultured with the GOEC monolayers *in vitro* in incubator at 39°C, 5% CO2 before transfer to recipients at GTC farm.

All experimental replicates for ATIII yielded cleavage stage embryos that were transferable on day 2 into synchronized recipients. Embryos using fibroblasts and epithelial cell phenotype as donor karyoplasts showed cleavage and development in culture. The percentage of cleavage development was higher in reconstructed couplets that used preactivated telophase II stage cytoplasts (45%) and telophase II-ethanol activated (56%) when compared to cytoplasts used at metaphase II arrested (35%) using ATIII karyoplasts. There were no differences observed in the cleavage rates of embryos that were reconstructed using donor karyoplasts in G₀, G₁ or S-phase of the cell cycle although, the morphological quality of embryos was better when donor karyoplasts were in as G₀ or G₁ compared to S-phase. Embryos were generally between the 2 to 8 cell stage with the majority of the embryos having 3-4 blastomeres at the time of transfer. Normal cleavage development corresponded chronologically to approximately 36 to 48 hours post fusion and activation. Morphologically normal appearing embryos were selected at the 2 to 8 cell stage following development in vitro for 36 to 48 hours.

### Estrus Synchronization of Recipient Does

Hormonal treatments were delayed by 1 day for recipients (as compared to donors) to insure donor/recipient synchrony. Estrus was synchronized on day 1 by a 6 mg subcutaneous norgestomet ear implant. A single injection of prostaglandin was administered on day 8. Starting on day 14, a single intramuscular treatment of PMSG (CalBiochem US) was administered. The ear implant was removed on day 15. Twenty-four hours following implant removal, recipient does were mated several times to vasectomized males over three consecutive days.

### Embryo Transfer to Recipient Does

Reconstructed embryos were co-cultured with GOEC monolayers for approximately 48 hours prior to transfer to synchronized recipients. Immediately-prior to transfer, reconstructed embryos were placed in equilibrated Ham's F-12 medium + 10% FBS. Two to four reconstructed embryos were transferred via the fimbria into the oviductal lumen of each recipient. Transfers were performed in a minimal volume of Hams's F-12 medium + 10% FBS using a sterile fire-polished glass micropipet.

The development of embryos reconstructed by nuclear transfer using transgenic caprine fetal fibroblasts and *in vivo* derived oocytes is summarized in Table 1. There was a total of 14 rounds of collection and transfers, with 4 donors set up for collection and 5-6 recipient does set up for transfer 48 hours later. The three different enucleationlactivation protocols were employed: Metaphase II, Telophase, and Metaphase II pretreated with Ethanol. Following fusion-activation, reconstructed embryos were co-cultured with primary goat epithelial cells, at least until cleavage (2-cell stage) up to early 16-cell stage; with most embryos being transferred at chronologically correct 2- and 4-cell stages. All transfers were surgical and oviductal, in hormonally synchronized recipients (due to the season). Rates of development were slightly superior when using the Telophase protocol and Ethanol protocol as compared to the Metaphase II protocol. This is partly due to the fact that enucleation of the second polar body seems less traumatic for the oocytes, and partly due to what seems to be higher activation rate for oocytes pretreated with ethanol.

**Table 1:**

| Development of caprine embryos reconstructed by nuclear transfer of transgenic fetal fibroblasts. Three enucleation/procedure were used: Metaphase II (first polar body enucleation), Telophase (second polar body enucleation), Ethanol (preactivation of Metaphase II stage oocytes by 7% ethanol treatment prior to enucleation). In all cases, concomitant fusion and activation was used. | | | | |
|---|---|---|---|---|
| Enucleation And activation protocol | Oocytes Reconstructed | Oocytes lysed (%) | Embryos Cleaved (%) | Embryos Transferred |
| Metaphase II | 138 | 67(48.5) | 48(35) | 47 |
| Telophase | 92 | 38(41) | 41(44) | 38 |
| Ethanol | 55 | 23(42) | 31(56) | 27 |

Following embryo transfer, recipient does were examined by ultrasound, as early as day 25. High pregnancy rates ranging from 55-78% for ATIII recipient does were diagnosed. For all three enucleation/activation protocols, it was observed that high proportion of does (65%) appeared positive at day 30. However, it must be noted that, in most cases, fetal heartbeats could not be detected at such an early stage. Moreover, the positive ultrasound signal detected at day 30 was not characteristic of normal embryo development and appeared closer to vesicular development not associated with the formation of an embryo proper. This kind of embryonic development is not typically observed in other caprine embryo transfer programs (for example with microinjected embryos). Biweekly, examination of these vesicular developments between day 25 and day 40 established that these pregnancies were abnormal and at day 40, most of the fetuses were reabsorbed and normal ultrasound images were not apparent.

However, for 2 pregnancies, heartbeats were detected by day 40. In these 2 cases, ultrasound examination between day 25 and day 40, not only detected a heartbeat, but also showed the development of recognizable embryonic structures. One of these pregnancies was established using the Metaphase II enucleation/activation protocol, fusing the enucleated cytoplast to a quiescent karyoplast originating from a passage 6 culture of the CFF 155-92-6 fibroblast cell line. In this instance, 4 four-cell stage reconstructed embryos were transferred to the oviduct of the recipient doe. The other pregnancy (twins) was obtained from embryos reconstructed according to the Telophase enucleation/activation protocol, fusing an enucleated cytoplast derived from preactivated telophase Ca²⁺ oocytes and G₁ karyoplasts originating from a passage 5 culture of the CFF155-92-6 epithelial cell line. In this case, 3 reconstructed embryos (1 two-cell stage and 2 four-cell stage) were transferred to the oviduct of the recipient doe.

No pregnancies were observed with embryos generated by the Ethanol enucleation/activation protocol. However, numbers are not large enough to conclude on the relative efficacy of the 3 enucleation/activation protocols used in this study.

**Table 2:**

| Induction of pregnancy and further development following transfer of caprine embryos reconstructed with transgenic fetal fibroblasts and activated according to three protocols | | | | | |
|---|---|---|---|---|---|
| Enucleation Activation protocol | Recipients (average # of embryos/ recip) | Ultrasound Results (positive/total recip) | | | Term pregnancies |
| | | 30 days | 40days | 50 days | |
| Metaphase II | 15(3.1) | 9/15 | 1/15 | 1/15 | 1 |
| Telophase | 14 (2 .7) | 11/14 | 1/14 | 1/14 | 1 (twins) |
| Ethanol | 9(3) | 5/9 | 0/9 | 0/9 | 0 |

### Perinatal Care of Recipient Embryos

Does were monitored daily throughout pregnancy for outward signs of health (e.g., appetite, alertness, appearance). Pregnancy was determined by ultrasonograph 25-28 days after the first day of standing estrus. Does were ultrasounded biweekly till approximately day 75 and there after once a month to monitor and assess fetal viability. Additionally, recipient does had serum samples drawn at approximately day 21 post standing estrus for serum progesterone analysis. This was to determine if a functioning corpus luteum was present and how this compared to the animal's reproductive status (i.e., pregnancy). At approximately day 130, the pregnant does were vaccinated with tetanus toxoid and Clostridium C&D. Selenium & vitamin E (Bo-Se) and vitamins A, D, and B complex were given intramuscularly or subcutaneously and a dewormer was administered. The does were moved to a clean kidding stall on approximately Day 143 and allowed to acclimate to this new environment prior to kidding. Observations of the pregnant does were increased to monitor for signs of pending parturition. After the beginning of regular contractions, the does remained under periodic observation until birth occurred. If labor was not progressive after approximately 15 minutes of strong contractions the fetal position was assessed by vaginal palpation. If the position appeared normal then the labor was allowed to proceed for an additional 5-30 minutes (depending on the doe) before initiating an assisted vaginal birth. If indicated a cesarean section was performed. When indicated, parturition was induced with approximately 5-10 mg of PGF2α (e.g. Lutalyse). This induction can occur approximately between 145-155 days of gestation. Parturition generally occurs between 30 and 40 hours after the first injection. The monitoring process is the same as described above.

Once a kid was born, the animal was quickly towel dried and checked for gross abnormalities and normal breathing. Kids were immediately removed from the dam. Once the animal was determined to be in good health, the umbilicus was dipped in 7% tincture of iodine. Within the first hour of birth, the kids received their first feeding of heat-treated colostrum. At the time of birth, kids received injections of selenium & vitamin E (Bo-Se) and vitamins A, D, and B complex to boost performance and health.

The first transgenic female goat offspring was produced by nuclear transfer was born after 154 days of gestation following the induction of parturition and cesarean delivery. The birth weight of the offspring was 2.35 kg which is within the medium weight range of the alpine breed. The female twins were born naturally with minimal assistance a month later with a gestation length of 151 days. The birth weights of the twins were both 3.5 kg which are also within the medium weight range for twins of this breed. All three kids appeared normal and healthy and were phenotypically similar for coat color and expressing markings typical of the alpine breed. In addition, all three offspring were similar in appearance to the transgenic founder buck. No distinguishable phenotypic influence from the breed of the donor oocyte (Saanen, Toggenburg breed) or the heterogeneous expression of the fetal genotype was observed.

### Transgenic Cloned Goats

In order to confirm that the three kids were transgenic for the BC6 construct comprising the goat beta casein promoter and the human ATIII gene sequence, PCR amplification and southern analysis of the segment of the transgene were performed.

Shortly after birth, blood samples and ear skin biopsies were obtained form the cloned female goats and the surrogate dams. The samples were subjected to genomic DNA isolation. Laird et al. (1991) *Nucleic Acids Res.* 19:4293. Each sample was first analyzed by PCR using AT IIl specific primers, and then subjected to Southern blot analysis using the AT III cDNA (Edmunds et al. (1998) *Blood* 91:4561-4571). For each sample, 5 µg of genomic DNA was digested with *Eco*RI (New England Biolabs, Beverly, MA), electrophoresed in 0.7% agarose gels (SeaKem®, ME) and immobilized on nylon membranes (MagnaGraph, MSI, Westboro, MA) by capillary transfer following standard procedures. Laird et al. (1991) *Nucleic Acids Res.* 19:4293. Membranes were probed with the 1.5 kb *Xho* I to *Sal* I AT III cDNA fragment labeled with α ⁻³²p dCTP using the Prime-It® kit (Stratagene, La Jolla, CA). Hybridization was executed at 65°C overnight. Church et al. (1984) *Prot. Natl Acad. Sci. USA* 81:1991-1995. The blot was washed with 0.2 X SSC, 0.1 % SDS and exposed to X-OMAT™ AR film for 48 hours.

PCR analysis confirmed that all of the kids were transgenic for the BC6 construct comprising the goat beta casein promoter and the human ATIII gene sequence. Southern blot analysis demonstrated the integrity of the BC6 transgene. Hybridization to a diagnostic 4.1 kb *EcoRI* fragment was detected for all three cloned animals, the cell lines and a transgenic positive control, but not for the two recipient does. As expected, due to cross hybridization of the ATIII cDNA probe to the endogenous goat AT locus, a 14 kb band was detected in all samples.

In addition, fluorescence *in situ* hybridization (FISH) was performed to determine the integration site of the BC6 construct. For typing of the cloned goats, whole blood was cultured for lymphocytes harvest. Ponce de Leon et al. (1992) *J. Hered.* 83:36-42. Fibroblast cells and lymphocytes were pretreated and hybridized as previously described in van de Corput et al. (1998) *Histochem Cell Biol.* 110:431-437, and Klinger et al. (1992) *Am. J Human. Genet.* 51:55-65. A digoxygen labeled probe containing the entire 14.7 kb BC6 transgene was used in this procedure. The TSA ™Direct system (NEN™ Life Science Products, Boston, MA) was used to amplify the signal. R-bands were visualized using DAPI counterstain and identified as in Di Berardino et al. (1987) *J. Hered.* 78:225-230. A Zeiss Axioskop microscope mounted with a Hamamatsu Digital Camera was used with Image-Pro ® Plus software (Media Cybernetics, Silver Spring, MD) to capture and process images.FISH analysis of blood cultures from each transgenic kid with probes for the BC6 transgene showed that all three carry a chromosome 5 transgene integration identical to that found in the metaphase plates derived from the CFF6 cell line. Moreover, analysis of at least 75 metaphase plates for each cloned offspring confirmed that they are not mosaic for the chromosome 5 transgenic integration.

As final confirmation that all three kids are derived from the transgenic CFF6 cell line, PCR-RFLP analysis for the very polymorphic MHC class II DRB gene was undertaken. Typing for the second exon of the caprine MHC class II DRB gene was performed using PCR-RFLP Typing as described Amills et al. (1996) *Immunopathol.* 55:255-260. Fifteen microliters of nested PCR product was digested with 20 units of *Rsal* (New England Biolabs, Beverly, MA). Following digestion, restriction fragments were separated at room temperature in a 4 to 20 % nondenaturing polycrylamide gel (MVP^{TM} precast gel, Stratagene, La Jolla, CA) in the presence of ethidium bromide.

As illustrated by the *Rsal* digests of the DRB gene second exon, the three cloned offspring are identical to each other and identical to the CFF6 donor cell line, whereas the recipient does carry different alleles.

### Induction of Lactation and Transgene Expression of Proteins in Milk

In order to determine whether the targeted mammary gland specific expression of human ATIII proteins were present in milk, the cloned transgenic prepubertal clones were transiently induced to lactate. At two months of age, the cloned offspring was subjected to a two week hormonal lactation-induction protocol. Hormonal induction of lactation for the CFF6-1 female was performed as described in Ryot et al. (1989) *Indian J. Anim. Res.* 10:49-51. The CFF6-1 kid was hand-milked once daily to collect milk samples for AT III expression analyses. All protein analysis methods were described in Edmunds et al. (1998) *Blood* 91:4561-4571. Concentration of recombinant ATIII in the milk was determined by a rapid reversephase HPLC method using a Hewlett Packard 1050 HPLC (Wilmington, DE) with detection at 214 nm. The ATIII activity was evaluated by measuring thrombin inhibition with a two-stage colorimetric endpoint assay. Western blot analysis was performed with an affinity purified sheep anti-ATIII HRP conjugated polyclonal antibody (SeroTec, Oxford, UK). Samples were boiled for 30 seconds in reducing sample buffer prior to loading onto a 10-20 % gradient gel (Owl Scientific). Electrophoresis was operated at 164 volts (constant) until the dye front ran off the gel.

At the end of the treatment, small milk samples of 0.5 to 10 ml were collected daily for 20 days. The small initial volumes of milk, 0.5 to 1 ml, were typical of the amounts seen in prepubertal female goats hormonally induced to lactate. The volumes increased to 10 ml per day by the time the female was dried off, 25 days after the onset. The concentration and activity of ATIII in several of the samples was evaluated. As previously noted with does from this specific BC6 transgenic cell line, high levels of the recombinant ATIII was detected by Western blot analysis. Edmunds et al. (1998) *Blood* 91:4561-4571. The concentration of recombinant ATIII in the milk of the cloned offspring was 5.8 grams per liter (20.5U/ml) at day 5, and 3.7 grams per liter (14.6 U/ml) by day 9. These were in line with levels recorded during the early part of a first natural lactation of does from this BC6 line (3.7 to 4.0 grams per liter).

### Discussion:

Healthy transgenic goats were obtained by nuclear transfer of somatic cells to oocytes that were enucleated either in the arrested Metaphase II or the activated Telophase II-stage. These studies show that serum-starved cells used to generate term pregnancies are likely at the G₀/ G₁ transition following restoration with 10% serum.

Immunofloresence screening revealed that after 7 days of serum starvation, fetal somatic cells were negative for G₁ stage cyclins D1, D2, D3 and PCNA; whereas within 3 hours of 10% FBS serum-activation a majority (e.g. approximately 70%) expressed these markers.

Reconstruction of an enucleated metaphase II arrested oocyte with the transfer of a nucleus from a donor karyoplast synchronized at G₀ or G₁ of the cell cycle following simultaneous fusion and activation mimic the chronological events occurring during fertilization. The successful development to term and birth of a normal and healthy transgenic offspring following the simultaneous fusion and activation protocol is in contrast with procedures employed in other studies that report the requirement for prolonged exposure of donor nuclei to elevated cytoplasmic MPF activity to support chromatin remodeling and reprogramming. *See* Campbell et al. (1996) *Nature* 380:64-66; Wilmut et al. (1997) *Nature* 385:810-813; Schnieke et al. (1997) *Science* 278:2130-2133; Cibelli et al. (1998) *Science* 280:1256-1258. This result challenges the contention that prolonged remodeling of the somatic nuclei in conditions of elevated MPF activity prior to activation is important for embryonic and fetal development to term. The results also demonstrate that a reconstructed embryo may not have a requirement for prolonged exposure of the donor nucleus to MPF nor are NEBD and PCC entirely requisite events. Rather chromatin remodeling events involving NEBD and PCC are likely permissive effects of MPF activity and, as such, may not be required for the acquisition of developmental competence or totipotency. Instead, these events are likely to serve to facilitate the acquisition of synchronicity between the cytoplast and the karyoplast. These events may even be detrimental if normal diploidy is not maintained when the donor nuclei are induced to undergo PCC with resultant chromosome dispersion due to an aberrant spindle apparatus due in part to MPF activity. Therefore, karyoplast and cytoplast synchronization with respect to cell cycle is important, first for maintenance of normal ploidy and, second for the proper induction of genome reactivation and subsequent acquisition of developmental competence of reconstructed embryos.

Further support is provided in the second method where chromatin-intact metaphase II arrested oocytes were activated to reduce MPF activity and induce the oocyte to exit the M-phase and enter the first mitotic cleavage. Approximately 3 hours post-activation, the oocytes were enucleated at telophase stage prior to the onset of G₁ and fused and simultaneously activated with a donor karyoplast in G₁ prior to START of the cycle. In addition, the simultaneous activation and fusion insured that tendencies of non-aged oocytes to revert back to an arrested state were circumvented. Using this paradigm, a normal and healthy set of twin cloned transgenic kids were produced. This procedure inherently provides a homogenous synchronization regimen for the cytoplast to coincide closer with the donor nuclei in G₁ prior to START. Further preactivation of the oocyte induces a decline in cytoplasmic MPF activity, thus inhibiting the occurrence of NEBD and PCC. These results suggest that NEBD and PCC is only facultative for the induction of cytoplast and karyoplast synchrony but not necessary for acquisition of proper genome reactivation and subsequent development to term of the nuclear transfer embryo using somatic cell nuclei. These findings further suggest that differentiated cells at the G₀ or G₁ stage function similar to embryonic blastomeres with respect to their ability to acquire totipotency when used in combination with an arrested or an activated recipient cytoplast. The use of both metaphase II arrested and telophase II cytoplasts provides dual options for cytoplast preparation in addition to providing an opportunity for a longer time frame to prepare the cytoplast. The use of Telophase II cytoplasts may have several practical and biological advantages. The telophase approach facilitates efficient enucleation avoiding the necessity for chromatin staining and ultraviolet localization. Moreover, enucleation at telophase enables removal of minimal cytoplasmic material and selection of a synchronous group of activated donor cytoplasts. This procedure also allows for the preparation of highly homogenous group of donor nuclei to be synchronized with the cell cycle of the cytoplast. When used for embryo reconstruction, these populations showed a higher rate of embryonic development *in vitro.* Thus, reconstructed embryos comprised of a synchronously activated cytoplast and karyoplast are developmentally competent.

In addition to a successful transgenic founder production, nuclear transfer of somatic cells allows for the selection of the appropriate transgenic cell line before the generation of cloned transgenic embryos. This is particularly important in the cases where several proteins are to be co-expressed by the transgenic mammary gland. For example, in the transgenic production of recombinant monoclonal antibodies in milk, heavy chain and light chain transgenes ideally should be expressed in the same secretory cells of the mammary epithelium at equivalent levels for the efficient production of intact antibodies. In addition, transgenes expressing each protein should be co-integrated in the same locus to favor equivalent expression and avoid segregation of heavy chain and light chain transgenes during herd propagation..

The generation of transgenic animals that have completely identical genetic backgrounds also enhances the possibility of studying the expression and secretion characteristics of recombinant proteins by the mammary gland. For example, the availability of several completely identical transgenic females producing recombinant human ATIII will help determine the extent of variation in the carbohydrate structure of this protein, as it is produced by the mammary gland. Thus, it may be feasible to improve the characteristics of the recombinant proteins produces in the transgenic animal system by varying environmental factors (e.g., nutrition) or to increase the milk volume yield of lactation-induction protocols to diminish further the time necessary to obtain adequate amounts of recombinant protein for pre-clinical or clinical programs.

The high-level expression of recombinant human ATIII detected in the milk of the CFF6-1 cloned goat illustrates one of the most important aspects of this technology. By combining nuclear transfer with lactation-induction in prepubertal goats, it may be possible to characterize transgenic animals and the proteins they secrete in 8 to 9 months from the time of cell line transfection of milk expression. The amount of milk collected in an induced lactation is not only sufficient to evaluate the recombinant protein yield, but, when mg per ml expression levels are obtained, is adequate for more qualitative analyses (glycosylation, preliminary pharmaco-kinetics, biological and pharmacological activities). The continued availability of the transfected donor cell line also insures that genetically identical animals can be quickly generated, to rapidly supply therapeutic proteins (with predictable characteristics) for clinical trials.

All patents and other references cited herein are hereby incorporated by reference.

Other embodiments are within the following claims:

SEQUENCE LISTING

<110> Genzyme Transgenics Corporation

<120> Transgenic and Cloned Animals

<130> EPP83923A

<140> Divisional of EP 1127113 derived from WO 00/26357
<141> 1999-11-02

<150> US 09/298,508
<151> 1999-04-22

<150> US 09/298,971
<151> 1999-04-23

<150> US 60/106,728
<151> 1998-11-02

<150> US 60/131,328
<151> 1999-04-26

<160> 4

<170> PatentIn version 3.2

<210> 1
<211> 28
<212> DNA
<213> Homo sapiens

<400> 1
ctccatcagt tgctggaggg tgtcatta 28

<210> 2
<211> 29
<212> DNA
<213> Homo sapiens

<400> 2
gaaggtttat cttttgtcct tgctgctca 29

<210> 3
<211> 24
<212> DNA
<213> Homo sapiens

<400> 3
ataatcacat ggagagccac aagc 24

<210> 4
<211> 24
<212> DNA
<213> Homo sapiens

<400> 4
gcacttcttt ggtatctgag aaag 24

## Claims

1. A method of producing a cloned non-human mammal comprising:
introducing a nucleus from a non-human mammalian somatic cell into a functionally enucleated oocyte, which is from the same species as said non-human somatic cell and which is in the metaphase II stage of meiotic cell division, to form a reconstructed embryo;
allowing said reconstructed embryo to develop into a mammal, thereby providing a cloned non-human mammal; and
wherein said nucleus from said non-human somatic cell further comprises at least one recombinant nucleic acid sequence under the control of at least one promoter sequence.

2. A method of making a transgenic non-human mammal comprising:
fusing a mammalian somatic cell capable of expressing a transgenic protein with a functionally enucleated oocyte, which is from the same species as the somatic cell and which is in the metaphase II stage of meiotic division, to obtain a reconstructed embryo;
activating the reconstructed embryo;
transferring the reconstructed embryo into a doe;
allowing the reconstructed embryo to develop into a mammal; and
wherein said nucleus from said mammalian somatic cell contains a recombinant nucleic acid sequence.

3. The method according to claim 1 or 2, wherein said at least one recombinant nucleic acid sequence is a DNA sequence encoding a desired gene that is actuated by a tissue specific promoter.

4. The method according to claim 3, wherein said tissue-specific promoter is a promoter preferentially expressed in mammary gland epithelial cells.

5. The method according to claim 4, wherein said promoter is selected from the group consisting of a β-casein promoter, a β-lactoglobin promoter, whey acid protein promoter and lactalbumin promoter.

6. The method according to claim 1 or 2, wherein said cloned non-human mammal is a goat.

7. The method according to claim 1 or 2, wherein said at least one recombinant nucleic acid sequence encodes a polypeptide selected from the group consisting of an α-1 proteinase inhibitor, an alkaline phosphotase, an angiogenin, an extracellular superoxide dismutase, a fibrogen, a glucocerebrosidase, a glutamate decarboxylase, a human serum albumin, a myelin basic protein, a pro-insulin, a soluble CD4, a lactoferrin, a lactoglobulin, a lysozyme, a lactoalbumin, an erytbropoietin, a tissue plasminogen activator, a human growth factor, an antitbrombin III, an insulin, a prolactin, and an α-1-antitrypsin.

8. The method according to claim 1 or 2, wherein said somatic cell is selected from a group of cell types present in a mammal including:
a) fibroblasts
b) cumulus cells
c) neural cells
d) mammary cells; and
e) myocytes.

9. The method according to claim 8, wherein the fibroblast is an embryonic fibroblast.

10. The method according to claim 1, wherein the somatic cell is in G₁ stage.

11. I The method according to claim 1, wherein the somatic cell is in G₀ stage.

12. The method according to claim 1 or 2, wherein the nucleus of the somatic cell is introduced into an enucleated oocyte by electrofusion.

13. The method according to claim 1 or 2, wherein the method further comprises mating the mammal which develops from the reconstructed embryo with a second mammal to produce a transgenic offspring.

14. The method according to claim 2, wherein the method further comprises mating the mammal which develops from the reconstructed embryo with a second mammal to produce a transgenic offspring.

15. The method according to claim 2, wherein said transgenic non-human mammal is a female mammal.

16. The method according to claim 15, wherein the mammal is induced to lactate.

17. The method according to claim 165wherein a product is recovered from the mammal.

18. The method according to claim 17, wherein a product is recovered from the milk, urine, hair, blood, skin, or meat of the mammal.

19. The method according to claim 17, wherein said product is a human protein.

20. The method according to claim 2, wherein the nucleus comprises a heterologous transgenic sequence under the control of a promoter.

21. The method according to claim 20, wherein the promoter is a caprine promoter.

22. The method according to claim 1 or 2, wherein said functionally enucleated oocyte is activated with ethanol.

23. The method according to claim 1 or 2, wherein said functionally enucleated oocyte is activated with a calcium ionophore.
